# EUROPEAN PATENT APPLICATION

(11) **EP 4 726 040 A1**
(43) Date of publication of application: **15.04.2026**
(21) Application number: 24383089.0
(22) Date of filing: 08.10.2024
(51) Int. Cl.: C12N 15/113, C12N 9/22

(54) **PSYCHROPHILIC AND THERMOLABILE CRISPR RIBONUCLEOPROTEIN COMPLEX AND THE USE THEREOF**

(71) Applicant: Consejo Superior de Investigaciones Científicas (CSIC), 28006 Madrid (ES); Fundació Institut d'Investigació Biomèdica de Bellvitge (IDIBELL), 08908 L'Hospitalet de Llobregat (Barcelona) (ES)
(72) Inventor: González Acinas, Silvia, 08003 Barcelona (ES); Cerón Madrigal, Julián, 08908 L Hospitalet de Llobregat (ES)
(74) Representative: San Martin Alarcia, Esther

(57) **Abstract**

The invention relates to a ribonucleoprotein complex comprising: a nuclease characterized as CRISPR-associated protein having a sequence identity of at least 70% to sequence SEQ ID NO: 1, and a dual or single guide RNA comprising a CRISPR RNA and a transactivating crRNA. Likewise, the present invention also relates to the use of said ribonucleoprotein to bind, modify or alter target sequences in the genome of a cell or organism, or to modify the gene expression of a cell.

## Description

The invention relates to a ribonucleoprotein complex. The ribonucleoprotein complex comprising: a nuclease characterized as CRISPR-associated (Cas) protein, and a guide RNA of the present invention to bind, modify or alter target sequences in the genome of a cell or organism, or to modify the gene expression of a cell. Thus, the present invention is within the molecular biology field.

### BACKGROUND ART

Recombinant DNA technology has made it possible to insert DNA sequences at targeted genomic locations and/or modify specific endogenous chromosomal sequences. Site-specific integration techniques, which employ site-specific recombination systems, as well as other types of recombination technologies, have been used to generate targeted insertions of genes of interest in a variety of organisms. Genome-editing techniques such as designer zinc finger nucleases (ZFNs), transcription activator-like effector nucleases (TALENs), or homing meganucleases, are available for producing targeted genome perturbations, but these systems tend to have low specificity and employ designed nucleases that need to be redesigned for each target site, which renders them costly and time-consuming to prepare.

Newer technologies utilizing archaeal or bacterial adaptive immunity systems have been identified, called CRISPR (Clustered Regularly Interspaced Short Palindromic Repeats), which comprise different domains of effector proteins that encompass a variety of activities (DNA recognition, binding, and optionally cleavage).

CRISPR-Cas systems include Cas proteins, which are involved in acquisition, targeting and cleavage of foreign DNA or RNA, and a guide RNA(s), which includes a segment that binds Cas proteins and a segment that binds to a target nucleic acid. The programmable nature of these systems has facilitated their use as a versatile technology for use in modification of target nucleic acid.

Despite the identification and characterization of some of these systems, there remains a need for CRISPR-Cas systems, with desirable properties such as psychrophilic or thermolabile. Further, expansion of the Cas9 nucleases toolbox with distinct enzymes presenting different properties is desirable to use the optimal Cas9 nuclease for each application of CRISPR-Cas technology.

### DESCRIPTION OF THE INVENTION

The authors of the present invention have developed a ribonucleoprotein complex comprising a guide RNA and an enzyme. The enzyme was isolated from deep ocean uncultured bacterium (bacteria not isolated in pure culture in laboratory conditions), identified by analysing deep ocean microbial metagenomes, with surprising distinctive biophysics properties, and structural and functional features. The isolated enzyme is a Cas9 nuclease which, surprisingly, shows psychrophilic properties, i.e. it presents good activity at low temperatures, even at 4°C, but more importantly it is easily denatured by moderate application of heat (thermolabile).

Likewise, the present invention also discloses the guide RNA specific for said nuclease and the ribonucleoprotein complex comprising thereof (Examples 1 and 2). The inventors of the present invention have observed that the nuclease, guide RNA and ribonucleoprotein complex disclosed herein, (i) provide activity in genome editing, such as editing of a target site on a polynucleotide sequence to a temperature as low as 4°C, and (ii) it is denaturalized above 38°C, losing its activity. In all ranges mentioned in the present description the extremes are included.

As can be seen, the examples of the present disclosure show the capacity of nuclease, guide RNA and ribonucleoprotein complex comprising them, of modifying a target nucleic acid sequence, specifically, cutting double-stranded DNA (dsDNA) in a PAM - dependent manner *in vitro* (Example 5), and *in vivo* in eukaryotic cells and organisms (Examples 4, 7 and 8).

After analysing the isolated Cas9 nuclease (also called "Deep Ocean 1 Cas9" or "DO1 Cas9"), the inventors have discovered that:
- DO1 Cas9 is a Class II Type II-C Cas9 of 991 amino acids, much smaller than canonical SpCas9 (1386 amino acids) (Class II Type II-A) from *Streptococcus pyogenes,* and therefore convenient for delivery systems.
- Coverage of microbial metagenomes assemblies and metagenomic assembled genomes (MAGs), upstream and downstream of the DO1 Cas9 gene, allowed the identification of crRNAs (repeats of the CRISPR system) and tracrRNA to infer the sequence for the guide RNA (gRNA).
- Upon *in vitro* translation (IVT), DO1 Cas9 showed high cleavage activity on plasmids with specific PAM sequences. Sequencing of the cleaved plasmids determined the PAM sequence for DO1 Cas9 as 5'-NRVDHCN-3' (according to IUPAC codes; N= any base, R= A>G, V= A or C or G, D= G>T>A, H=C>T>A, C= Cytosine, N=G>T or A>C). The DO1 Cas9 cut site is 3bp upstream of the PAM site and leaves blunt ends.
- DO1 Cas9 displays low activity at 37°C in cultured human cells, using DO1 Cas9 plasmids for transfection or DO1 Cas9 Ribonucleoproteins (RNPs).
- DO1 Cas9 protein is thermolabile. In vitro, its nuclease activity is negatively affected by preincubating the protein at 33°C for 5 minutes and completely inactivated when incubated for 15 minutes. It can be also inactivated at lower temperatures with longer incubation periods, i.e. 31°C temperature for 30 minutes. Once DO1 Cas9 is coupled with the gRNA, the thermostability of the RNPs is higher than the protein alone. DO1 Cas9 ribonucleoprotein complex showed an optimal temperature range for nuclease activity in vitro between 10°C and 35°C, with some activity at 4°C, and low activity at 37°C and null activity at 39°C.
- DO1 Cas9, besides being heat-labile, presents features of cold-adapted or psychrophilic enzymes such as a flexible protein structure. Moreover, in DO1 Cas9, activation energy is lower, and denaturation transition is slower, than in SpCas9 as determined by circular dichroism and fluorescent spectroscopy.
- DO1 Cas9 is suitable and efficient for genome editing *in vivo* at mild temperatures (15-28°C at least) (about 35-40% efficiency in the nematode *Caenorhabditis elegans* and in the vertebrate zebrafish). As expected from *in vitro* experiments, DO1 Cas9 cuts dsDNA *in vivo* 3bp upstream of the PAM site.
- Modeling DO1 Cas9 by AlphaFold allows the identification of catalytic residues that can be mutated to produce nickase and dead versions of the protein.

In view of the foregoing, several inventive aspects have been developed which are disclosed in detailed below.

### Ribonucleoprotein complex of the invention

In one aspect, the present invention provides a ribonucleoprotein complex, hereinafter "ribonucleoprotein complex of the invention", comprising:
(i) a nuclease (hereinafter "nuclease of the ribonucleoprotein complex of the invention"), wherein the nuclease comprises an amino acid sequence having a sequence identity of at least 70%, at least 75%, at least 80%, at least 85%, at least 90%, at least 95%, at least 98%, at least 99% or 100% to SEQ ID NO: 1; and
(ii) a guide RNA (hereinafter "guide RNA of the ribonucleoprotein complex of the invention"), wherein the guide RNA is a dual or single guide RNA comprising:
   a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3, and
   b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4.

In the present invention, "ribonucleoprotein complex" refers to a guide RNA and a nuclease that are capable of forming a complex, wherein said complex can direct the nuclease to a DNA target site, enabling nuclease to recognize, bind to, and optionally nick or cleave (introduce a single or double-strand break) the DNA target site. These complexes play an integral part in several important biological functions that include transcription, translation and regulating gene expression and regulating the metabolism of RNA The ribonucleoprotein complex of the invention is a ribonucleoprotein that comprises a nuclease and a guide RNA. Thus, when the ribonucleoprotein complex of the invention is active, it binds and cleaves DNA. However, when the ribonucleoprotein complex is inactive, it binds to DNA but does not cut it. The ribonucleoprotein complex can be inactivated by locating and modifying the catalytic amino acid of the nuclease (Example 9) and denatured by applying heat above 38°C to the nuclease.

The ribonucleoprotein complex of the invention comprises a (i) nuclease and a (ii) RNA guide, wherein the nuclease comprises an amino acid sequence having a sequence identity of at least 70%, at least 75%, at least 80%, at least 5%, at least 90%, at least 95%, at least 98%, at least 99% or 100% to SEQ ID NO: 1. In a particular embodiment, the ribonucleoprotein complex of the invention comprises the nuclease comprising an amino acid sequence with a sequence identity of 100% to SEQ ID NO: 1.

In a more particular embodiment, the nuclease of the ribonucleoprotein complex of the invention consists of an amino acid sequence having a sequence identity of at least 70%, at least 75%, at least 80%, at least 85%, at least 90%, at least 95%, at least 98%, at least 99%, at least 99.1 %, at least 99.2%, at least 99.3%, at least 99.4%, at least 99.5%, at least 99.6%, at least 99.7%, at least 99.8%, or at least 99.9% to SEQ ID NO: 1. In another even more particular embodiment, the nuclease of the ribonucleoprotein complex of the invention consists of an amino acid sequence with a sequence identity of 100% to SEQ ID NO: 1.

SEQ ID NO: 1:
MTKKVLSIDPGVTSIGWTLTEQNEKGHLTKFIDGGSRIFNSVKEAKSGTFKNEKRRA
KRTTRRNKYRSNRRMAKLENYLIKNDFLSNKISSLSDHTGLVQQIIGNPYHLRKKAL
YEPLTNEELAWAIIHLAKRRGFLSSSKSLTPEEKKEQARADELATLMKKEKCKTLGE
YFSNLYDKGEKIRGEKLKRQLIQDELELILNKQMETNSLITYKFVNDINTIIFNQRPLK
TQKRISLCKFESFGKIKKHTAAKLHPLSQQFILWGFINNLKYNDNSGGMQELTLEQK
NVVFDLLWTNKEVTFKKIIKELKLTPETTFNFQKGEDDKVKGNQLLNYFKAKTKTWF
NSLSEQQKIDLIQDLSTINDLTGFALKKRIYNHWTKDEEIIENLLLTSDKLPQGYTSLS
LKATKKILPFLKAGNKYYEAEKKAYPNFTINTKKVNKLPPVEGISNTVVLKSCSQVR
HLVNAIVDKYGEIDIIKIELARDLSKSKKQISETNILNSKNKSLNDEAISYLHKNNIDVN

HDNITKYKLWKESESSDKQHRSCYPEQDSDGNWFFPTINLNDLYGANAIYEIEHIIP
KSISGDDSFANKALCKHKTNAQKGNKTPYDYYFQKGGQELIDMIAKTAYSQFGKW
KGKKFVTSTEKYLDENDIQKRYLNDTRYLAVFLKEYLQPVCKDKIVVSKGGFTAIIR
QSLNLNILLGNEFNKNRGDYRHHMVDAFCTSLISPKVLDIINIMRKQFNKKTEKYKE
CETKLNDYIKNVKNDFIKHFDNVITSHEVESKLKGELEEETLYGHEKIKLDDGSIITKL
FQHKPYEEVISKYNSPEEVLKAHDNLEISLPKGLKKYLENNNKLPDMFQGWKRIKK
YYNNIDENNFNINIYRAIHNEKEKVIAYKKTQENAYIIVYNDFKNETISKMNEISNKPK
FNNVFCKLYKGDLIQDSENNIYKIYQLLSDKRIALHPVNIQPIKTKRKSVKLIFDKEKM
YKIKVNILGHII

The term of "nuclease" is used herein to mean an enzyme which possesses catalytic activity for nucleic acid cleavage [e.g., ribonuclease activity (ribonucleic acid (RNA) cleavage), or deoxyribonuclease activity (deoxyribonucleic acid (DNA) cleavage), etc.]. Nucleases are called endonucleases if they are capable of cutting the phosphodiester bond in nucleotides located within the nucleic acid chain, and they are called exonucleases if they cut the phosphodiester bonds of the nucleotides at the ends of the chains.

In the present invention, by "cleavage" or "cut" is meant the breakage of the covalent backbone of a target nucleic acid molecule (e.g., RNA, DNA). Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events.

The nuclease of the ribonucleoprotein complex of the invention comprises an amino acid sequence having a sequence identity of, at least, 70% to SEQ ID NO: 1. As use herein, the expression "sequence identity" refers to the extent to which two sequences (nucleotide or amino acid) have the same residue at the same positions in an alignment. For example, "an amino acid sequence has a sequence identity of X% to SEQ ID NO: Y" refers to the X% of residues that are identical between respective positions of two sequences when the two sequences are aligned for maximum sequence identity. The % identity is calculated by dividing the total number of the aligned residues by the number of the residues that are identical between the respective positions of at least two sequences and multiplying by 100. Generally, computer programs can be employed for such calculations. Examples of Illustrative programs that compare and align pairs of sequences, include, but are not limited to, ALIGN, gapped BLAST, BLASTP, BLASTN, T-COFFEE, MUSCLE, MAFFT, etc.

As explained in the beginning of the present disclosure, the nuclease of the ribonucleoprotein complex of the invention was isolated from deep ocean uncultured bacteria.

The nuclease of the ribonucleoprotein complex of the invention is a Cas protein. The term "Cas" refers herein to a polypeptide encoded by a Cas (CRISPR- associated) gene. "CRISPR" (Clustered Regularly Interspaced Short Palindromic Repeats) loci refers to certain genetic loci encoding components of DNA cleavage systems, for example, used by bacterial and archaeal cells to destroy foreign DNA. A CRISPR locus can consist of a CRISPR array, comprising short direct repeats (CRISPR repeats) separated by short variable DNA sequences (called spacers), which can be flanked by diverse Cas (CRISPR-associated) genes. In another particular embodiment, alone or in combination with the above particular embodiments of the ribonucleoprotein complex of the invention, the nuclease is a Cas9 protein.

As used herein, a "Cas9 protein" or "Cas9" is a Cas endonuclease that forms a guide RNA with a CRISPR RNA (crRNA) and a transactivating crRNA (tracrRNA) for specifically recognizing, binding and cleaving all or part of a DNA target sequence. Cas9 recognizes a 3' GC-rich PAM sequence on the target dsDNA. A Cas9 protein comprises a RuvC nuclease with an HNH (H-N-H) nuclease adjacent to the RuvC-II domain. The RuvC nuclease and HNH nuclease each can cleave a single DNA strand at a target sequence (the concerted action of both domains leads to DNA double-strand cleavage, whereas the activity of one domain leads to a nick). In general, the RuvC domain comprises subdomains I, II and III, where domain I is located near the N-terminus of Cas9 and subdomains II and III are located in the middle of the protein, flanking the HNH domain. Cas9 endonucleases are typically derived from a type II CRISPR system, which includes a DNA cleavage system utilizing a Cas9 endonuclease in complex with at least one polynucleotide component. For example, a Cas9 can be in complex with a CRISPR RNA (crRNA) and a trans-activating CRISPR RNA (tracrRNA). In another example, a Cas9 can be in complex with a single guide RNA. Thus, in another particular embodiment of the ribonucleoprotein complex of the invention, alone or in combination with each and every one of the previous particular embodiments, the nuclease is a Cas 9 of class II, still more preferably, class II-C.

The nuclease of the ribonucleoprotein complex of the invention shows psychrophilic properties, and it is easily inactive by moderate application of heat (thermolabile).

As used herein, "Psychrophilic enzymes", also called "Cold-adapted enzymes", are enzymes that can effectively catalyze low temperature reactions, as low as 0°C, and are very temperature sensitive. Thus, it is expected that psychrophilic enzymes, such as the nuclease of the invention, have greater structural flexibility at active sites to increase the catalytic rate constant, and a higher degree of conformational complementarity with substrates to allow a decrease in activation energy when compared to mesophilic and thermophilic homologs. In other words, cold-adapted enzymes display high catalytic rates at low temperatures and are most frequently, but not always, characterized by an increased flexibility and reduced stability and substrate affinity.

The nuclease of the ribonucleoprotein complex of the invention can be used within a broad temperature range from a low temperature of 0°C to a high temperature of about 38°C. Since the nuclease of the ribonucleoprotein complex of the present invention can show its activity within a broad temperature range, those skilled in the art can use it under various temperature conditions in function of the intended purpose. For example, the nuclease of the ribonucleoprotein complex of the invention can carry out nucleic acid digestion under constant temperature conditions, under variable temperature conditions (within a certain range), or under predetermined or programmed temperature cycling conditions and the like. Particularly, the nuclease of the ribonucleoprotein complex of the invention may be enzymatically active, or has detectable nuclease activity, in a temperature range of from 0°C to 38°C. In some cases, the nuclease of the ribonucleoprotein complex of the invention is enzymatically active, or has detectable nuclease activity, in a temperature range of from 4°C to 35°C. In some cases, the nuclease of the ribonucleoprotein complex of the invention is enzymatically active, or has detectable optimal nuclease activity, in a temperature range of from 10°C to 30° C. In some cases, the nuclease of the ribonucleoprotein complex of the invention is enzymatically active, or has detectable optimal nuclease activity, in a temperature range of from 15°C to 25°C. In some cases, the nuclease of the ribonucleoprotein complex of present invention exhibits maximal enzymatic activity or has detectable nuclease activity (as defined by cleavage rate of a target nucleic acid when complexed with a guide RNA (gRNA) in a temperature range of from 10°C to 35 °C. Thus, in another particular embodiment of the nuclease of the ribonucleoprotein complex of the invention, alone or in combination with each and every one of the above particular embodiments, the nuclease is enzymatically active (or has detectable nuclease activity) in a temperature range of from 0°C to 38°C, particularly 15°C to 35°C, more particularly at 20°C, still more particularly at 4°C. In all ranges mentioned in the present description the extremes are included.

Additionally, the nuclease of the ribonucleoprotein complex of the invention may be inactivated by moderate application of heat at temperatures over 31°C (after 15 minutes at 33°C or 30 minutes at 31°C), thereby reducing or eliminating its nuclease activity. In addition, it may be dramatically affected by temperature with a drop in the activity around 37°C compared to milder temperatures as 25°C. The term "thermolabile" refers herein to an enzyme readily destroyed or deactivated by heat. Thus, in another particular embodiment of the nuclease of the ribonucleoprotein complex of the invention, alone or in combination with each and every one of the above particular embodiments, the nuclease is enzymatically inactive in a temperature above 38°C. In all ranges mentioned in the present description the extremes are included.

The terms "enzymatic activity", "enzymatically active" and "nuclease activity" as used herein with reference to a nuclease of the ribonucleoprotein complex of the invention refers to cleavage of one or both strands of a nucleic acid target by the protein.

The terms "target nucleic acid", "target site", "target sequence" or "target DNA" are used interchangeably herein and refer to a polynucleotide sequence in the genome (including choloroplastic and mitochondrial DNA) of a cell at which a double-strand break (or a single strand if the nuclease is engineered to present nickase activity), is induced in the cell genome by a nuclease of the invention. The target nucleic acid is the sequence to which the guide sequence of a nuclease gRNA (e.g., a dual gRNA or a single-molecule gRNA) will hybridize. For example, the target sequence 5'-GAGCATATC-3' within a target nucleic acid is targeted by, or is bound by, or hybridizes with, or is complementary to, the sequence 3'-CUCGUAUAG-5'. Suitable hybridization conditions include physiological conditions normally present in a cell. For a double-stranded target nucleic acid, the strand of the target nucleic acid that is complementary to and hybridizes with the gRNA is referred to as the "complementary strand" or "target strand"; while the strand of the target nucleic acid that is complementary to the "target strand" (and is therefore not complementary to the gRNA) is referred to as the "non-target strand" or "non- complementary strand."

The nuclease of the ribonucleoprotein complex of the invention may be capable of being guided by a gRNA to target single or double-stranded DNA in a protospacer adjacent motif (PAM)-dependent manner (protospacer adjacent motif). As is the case for many CRISPR endonucleases, such as Cas9, site-specific binding (and/or excision) of a double-stranded target DNA occurs at locations determined by (i) base pairing complementarity between the guide RNA and the target DNA; and (ii) a short motif PAM in the target DNA. The Cas endonuclease may not successfully recognise a target DNA sequence if the target DNA sequence is not followed by a PAM sequence. The PAM sequence can be any length, but is typically 1, 2, 3, 4, 5, 6, 7, 8, 9, 10, 11, 12, 13, 14, 15, 16, 17, 18, 19 or 20 nucleotides long. The term "binding", as used herein (e.g. with reference to an RNA -binding domain of a polypeptide, binding to a target nucleic acid, and the like), refers to a non-covalent interaction between macromolecules (e.g., between a protein and a nucleic acid; between a ribonucleoprotein complex (nuclease and guide RNA) and a target nucleic acid; and the like).

The term "protospacer adjacent motif" or "PAM" as used herein, refers to a short nucleotide sequence adjacent to a target sequence (protospacer) that is recognised (targeted) by a ribonucleoprotein complex that comprises a guide RNA and nuclease of the invention. In another particular embodiment of the ribonucleoprotein complex of the invention, alone or in combination with each and every one of the above particular embodiments, the nuclease binds and cleaves the target polynucleotide in a protospacer adjacent motif (PAM) - dependent manner. In another particular embodiment of the ribonucleoprotein complex of the invention, alone or in combination with each and every one of the above particular embodiments, the nuclease cleaves a double-stranded target polynucleotide at 3 base pairs upstream of PAM sequence leaving blunt ends, or a single-stranded target polynucleotide at 3 base pairs upstream of PAM sequence. In another particular embodiment of the ribonucleoprotein complex of the invention, alone or in combination with each and every one of the above particular embodiments, the PAM sequence comprises the sequence NRVDHCN wherein N is any base, R is A or G, V is A or C or G, D is G or T or A, H is C or T or A, C is Cytosine, N is any base.

The ribonucleoprotein of the invention may be capable of being guided by a guide RNA to target single or double-stranded DNA in a PAM-dependent manner. Thus, the ribonucleoprotein comprises a (i) nuclease and a (ii) RNA guide, wherein the RNA guide is:
- A dual guide RNA comprising:
   a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3, and
   b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4;
      or
- A single guide RNA comprising a nucleotide sequence having a sequence identity of at least 70%, at least 75%, at least 80%, at least 85%, at least 90%, at least 95%, at least 98%, at least 99% or 100% to SEQ ID NO: 5.

As used herein, the term "guide RNA" or "gRNA", relates to a polynucleotide sequence that solely comprises ribonucleic acids, and that can form a ribonucleoprotein complex with a protein, such as the nuclease described herein, and enables the protein to recognize, optionally bind to, and optionally cleave, a DNA target site.

The naturally existing guide RNA comprises two separate molecules, the tracrRNA (trans-acting CRISPR RNA) and the crRNA (CRISPR RNA), that can hybridize to form a "dual guide RNA" or "dgRNA". The gRNA can also be synthetically synthesized as a unique molecule containing both sequences of the tracrRNA and the crRNA which is referred as "single guide RNA" or "sgRNA".

On the one hand, the ribonucleoprotein complex of the invention comprises a dual guide RNA hereinafter "dgRNA of the ribonucleoprotein of the invention" or a single guide RNA hereinafter "sgRNA of the ribonucleoprotein of the invention", comprising:
a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3,
b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4.

SEQ ID NO: 3, nucleotide repeat sequence of a crRNA, direction 5' to 3':
AUUGUAAUUAGUUAACAUUUAUUAUAUACUUAAAAU

SEQ ID NO: 4, nucleotide sequence of a tracrRNA, direction 3' to 5':

UUUCUUUUUUCGGAUAACUUUAGCUCAAAGUUAUCUGAUAAAUAUCAAAUUU
UGAAUGCUGUUGUUACAACAGAAUAACAUUAUGUGAUCAAUUGUAAAUAAUA
UAUGAAU

In a particular embodiment of the ribonucleoprotein complex of the invention, alone or in combination with each and every one of the previous particular embodiments, the nucleotide sequence of crRNA of the dgRNA consists of the sequence SEQ ID NO: 3. In a particular embodiment of the ribonucleoprotein complex of the invention, alone or in combination with each and every one of the previous particular embodiments, the nucleotide sequence of tracrRNA of dgRNA consists of the sequence SEQ ID NO: 4.

The ribonucleoprotein complex of the invention comprises a single guide RNA, wherein the crRNA and the tracrRNA may be bound by a linker. The linker of a single guide RNA described in the present description is a stretch of nucleotides, it can have a length of at least 3 or 4 nucleotides. Preferably, the linker of a single guide RNA is GAAA.

Thus, in a particular embodiment, the single guide RNA comprises a nucleotide sequence having a sequence identity of at least 70%, at least 75%, at least 80%, at least 85%, at least 90%, at least 95%, at least 98%, at least 99% or 100% to SEQ ID NO: 5.

SEQ ID NO: 5, nucleotide sequence of a single guide RNA (direction 5' to 3'):

AUUGUAAUUAGUUAACGAAAGUUAACUAGUGUAUUACAAUAAGACAACAUUG
UUGUCGUAAGUUUUAAACUAUAAAUAGUCUAUUGAAACUCGAUUUCAAUAGG
CUUUUUUCUUU

The term "single guide RNA" or "sgRNA" relates to a synthetic fusion of two RNA molecules, a crRNA comprising a variable targeting domain (linked to a tracr mate sequence that hybridizes to a tracrRNA), fused to a tracrRNA.

In a particular embodiment of the sgRNA of the ribonucleoprotein complex of the invention, the nucleotide sequence of the sgRNA consists of the sequence SEQ ID NO: 5.

In a particular embodiment of the ribonucleoprotein complex of the invention, the guide RNA recognises a target nucleic acid sequence in a single or a double-stranded target polynucleotide.

In another particular embodiment of the ribonucleoprotein complex of the invention, the guide RNA described herein is substantially complementary along its length to a target DNA sequence. In another particular embodiment of the ribonucleoprotein complex of the invention, alone or in combination with each and every one of the above particular embodiments, the single or double-stranded target polynucleotide comprising the target nucleic acid sequence is cleaved by the nuclease described herein, preferably wherein said cleavage is DNA cleavage.

The target polynucleotide may further comprise a PAM sequence directly adjacent the 3' end of a protospacer sequence, in accordance with a PAM sequence provided herein. The guide RNA may be bound to or associated with the target sequence within the ribonucleoprotein complex, so that the target polynucleotide, comprising the target sequence and PAM sequence on the non-target strand, may be associated with and so form part of a ribonucleoprotein complex of the invention.

Alteration of the sequence of the guide RNA which associates with the nuclease of the invention therefore allows the nuclease to be programmed to mark or cut single or double-stranded DNA at sites complementary to the guide RNA.

### Guide RNA (gRNA) of the invention

The present invention also encompasses a guide RNA useful to lead a nuclease to specifically modify or alter target sequences in the genome of a cell or organism. This guide RNA may be a dual guide RNA or a single guide RNA.

Thus, in another aspect, the present invention relates to a dual guide RNA, hereinafter "dgRNA of the invention", comprising:
a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3,
b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4.

In a particular embodiment of the dgRNA of the invention, alone or in combination with each and every one of the previous particular embodiments, the nucleotide sequence of crRNA consists of the sequence SEQ ID NO: 3. In a particular embodiment of the dgRNA of the invention, alone or in combination with each and every one of the previous particular embodiments, the nucleotide sequence of tracrRNA consists of the sequence SEQ ID NO: 4.

In another aspect, the present invention relates to a single guide RNA, hereinafter "sgRNA of the invention", comprising a nucleotide sequence having a sequence identity of at least 70%, at least 75%, at least 80%, at least 85%, at least 90%, at least 95%, at least 98%, at least 99% or 100% to SEQ ID NO: 5.

The term "single guide RNA" or "sgRNA" relates to a synthetic fusion of two RNA molecules, a crRNA comprising a variable targeting domain (linked to a tracr mate sequence that hybridizes to a tracrRNA), fused to a tracrRNA.

In a particular embodiment of the sgRNA of the invention, the nucleotide sequence of the sgRNA consists of the sequence SEQ ID NO: 5.

In some embodiments, there is a linker polynucleotide that connects the crRNA and tracrRNA to form the single guide RNA. The linker of a single guide RNA described in the present description is a stretch of nucleotides, it can have a length of at least 3 nucleotides. Preferably, the linker of a single guide RNA is GAAA.

The terms "sequence identity", "guide RNA", "dual guide RNA", "single guide RNA", "crRNA sequence" and "a tracrRNA sequence" have been disclosed above for the previous inventive aspect and their definitions and their particular embodiments are applicable to the present inventive aspect.

### Nuclease of the invention

The present invention also relates to a nuclease.

Thus, in another aspect, the present invention refers to a nuclease, hereinafter "nuclease of the invention", comprising an amino acid sequence having a sequence identity of at least 70%, at least 75%, at least 80%, at least 85%, at least 90%, at least 95%, at least 98%, at least 99%, at least 99.1%, at least 99.2%, at least 99.3%, at least 99.4%, at least 99.5%, at least 99.6%, at least 99.7%, at least 99.8%, or at least 99.9% to SEQ ID NO: 1. In a more particular embodiment, the nuclease of the invention comprises an amino acid sequence with a sequence identity of 100% to SEQ ID NO: 1.

In an even more particular embodiment, the nuclease of the invention consists of an amino acid sequence having a sequence identity of at least 70%, at least 75%, at least 80%, at least 85%, at least 90%, at least 95%, at least 98%, at least 99%, at least 99.1%, at least 99.2%, at least 99.3%, at least 99.4%, at least 99.5%, at least 99.6%, at least 99.7%, at least 99.8%, or at least 99.9% to SEQ ID NO: 1. In another even more particular embodiment, the nuclease of the invention consists of an amino acid sequence with a sequence identity of 100% to SEQ ID NO: 1.

The term "sequence identity" has been defined previously in the present description. Likewise, all the particular embodiments and definitions disclosed in previous paragraphs for the nuclease of the ribonucleoprotein complex of the invention are applicable to the present inventive aspect.

As explained at the beginning of the present disclosure, the nuclease of the invention was isolated from deep ocean uncultured bacteria.

The nuclease of the invention is a Cas protein, specifically a Cas 9 protein, and as indicated above this kind of protein presents endonuclease activity. Thus, in a particular embodiment, alone or in combination with the above particular embodiments, the nuclease of the invention is a Cas9 protein. In another particular embodiment of the nuclease of the invention, alone or in combination with each and every one of the previous particular embodiments, the nuclease of the invention is a Cas 9 of class II, still more preferably, class II-C.

The nuclease of the invention shows psychrophilic properties, that is, it can effectively catalyze low temperature reactions, and it is easily denatured by moderate application of heat (thermolabile), which is irreversible. Thus, it is expected that psychrophilic enzymes, such as the nuclease of the invention, have greater structural flexibility to increase the catalytic rate constant, and a higher degree of conformational complementarity with substrates to allow a decrease in activation energy when compared to mesophilic and thermophilic homologs.

The nuclease of the invention can be used within a broad temperature range of from a low temperature of 0°C to a high temperature of about 38°C. Since the nuclease of invention can show its activity within a broad temperature range, those skilled in the art can use it under various temperature conditions in function of the intended purpose. For example, the nuclease of the invention can carry out nucleic acid digestion under constant temperature conditions, under variable temperature conditions (within a certain range), or under predetermined or programmed temperature cycling conditions and the like. Particularly, the nuclease of the invention may be enzymatically active, or has detectable nuclease activity, in a temperature range of from 0°C to 38°C. In some cases, the nuclease of the invention is enzymatically active, or has detectable nuclease activity, in a temperature range of from 4°C to 35°C. In some cases, the nuclease of the invention is enzymatically active, or has detectable optimal nuclease activity, in a temperature range of from 10°C to 30° C. In some cases, the nuclease of the invention is enzymatically active, or has detectable optimal nuclease activity, in a temperature range of from 15°C to 25°C. In some cases, the nuclease of the present invention exhibits maximal enzymatic activity or has detectable nuclease activity (as defined by cleavage rate of a target nucleic acid when complexed with a guide RNA (gRNA)) in a temperature range of from 10°C to 35 °C. Thus, in another particular embodiment of the nuclease of the invention, alone or in combination with each and every one of the above particular embodiments, the nuclease is enzymatically active (or has detectable nuclease activity) in a temperature range of from 0°C to 38°C, more at 4°C. In all ranges mentioned in the present description the extremes are included.

Additionally, the nuclease of the invention may be dramatically affected by temperature (i.e. it is a thermolabile enzyme) with a drop in the activity around 37°C compared to milder temperatures as 25°C. In the absence of gRNA, the nuclease is even more thermolabile and may be inactivated by moderate application of heat at temperatures over 31°C (after 15 minutes at 33°C or 30 minutes at 31°C). Thus, in another particular embodiment of the nuclease of the invention, alone or in combination with each and every one of the above particular embodiments, the nuclease is inactive in a temperature above 38°C. In another particular embodiment of the nuclease of the invention, the nuclease is denatured after 15 minutes at 33°C. In all intervals mentioned in the present description the extremes are included.

The term "denatured" refers to the destruction of the characteristic properties of a protein by heat, acidity, or other effect which disrupts its molecular conformation. As use herein, the nuclease of the invention is denatured by heat above 38°C, altering its standard three-dimensional structure, and rendering the nuclease non-functional, that is, it cannot exert its activity.

### Polynucleotide of the invention

The present invention also encompasses a polynucleotide comprising a nucleotide sequence encoding the nuclease of the invention. Thus, in another aspect, the present invention refers to a polynucleotide, hereinafter "polynucleotide of the invention", comprising a nucleotide sequence encoding the nuclease of the invention.

The terms "polynucleotide" and "nucleic acid," used interchangeably herein, refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA -RNA hybrids, or a polymer comprising purine and pyrimidine bases or other natural, chemically or biochemically modified, non-natural, or derivatized nucleotide bases.

Alternatively, in another particular embodiment of the polynucleotide of the invention, alone or in combination with each and every one of the previous particular embodiments, the polynucleotide consists of a nucleotide sequence encoding the nuclease of the invention.

In another particular embodiment of the polynucleotide of the invention, alone or in combination with each and every one of the previous particular embodiments, the nucleotide sequence has an sequence identity of, at least 70%, at least 71%, at least 72, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% to SEQ ID NO: 2. The expression "sequence identity" has been defined above in previous paragraphs. In more particular embodiment of the polynucleotide of the invention, the nucleotide sequence has a sequence identity of 100% to SEQ ID NO: 2.

SEQ ID NO: 2:

ATGACAAAAAAAGTATTATCAATAGACCCTGGCGTTACCTCTATCGGATGGACA
TTAACAGAACAAAATGAAAAAGGACACTTAACTAAATTCATTGATGGCGGTTCA
AGAATTTTCAACTCTGTAAAAGAAGCTAAAAGTGGTACATTTAAAAATGAAAAAA
GAAGAGCTAAAAGAACAACCAGAAGGAATAAATACAGATCTAATCGAAGAATGG
CTAAGTTAGAAAACTATTTAATTAAAAATGACTTCTTATCTAACAAAATAAGCTCT
CTTTCTGATCATACTGGACTTGTTCAACAAATTATAGGTAATCCTTACCATTTAA
GAAAAAAAGCACTTTACGAACCATTAACTAACGAAGAATTAGCTTGGGCTATTA
TTCATTTAGCAAAAAGAAGAGGTTTCTTATCAAGCTCTAAATCATTAACACCAGA
AGAAAAGAAAGAACAGGCAAGAGCCGATGAATTAGCTACTCTTATGAAAAAAGA
AAAATGTAAAACACTTGGTGAATACTTTTCTAATCTATATGATAAAGGTGAAAAA
ATACGTGGTGAAAAATTAAAAAGACAACTTATTCAAGATGAATTAGAATTAATCT
TAAATAAACAAATGGAAACTAATTCTTTAATTACTTATAAATTTGTTAATGATATT
AATACTATTATTTTTAATCAAAGACCACTTAAAACTCAAAAAAGAATTTCTCTATG
TAAATTTGAATCTTTTGGTAAAATAAAAAAACATACTGCGGCTAAACTTCATCCT
TTAAGTCAACAATTTATTCTTTGGGGATTTATTAATAATTTAAAATATAATGATAA
CTCTGGCGGCATGCAAGAATTAACATTAGAACAAAAAAATGTTGTCTTTGATTTA
TTGTGGACAAATAAAGAAGTTACCTTTAAAAAAATTATTAAAGAACTAAAATTAA
CGCCTGAAACTACTTTTAATTTTCAAAAAGGTGAAGATGATAAAGTAAAAGGGA
ATCAATTACTCAATTACTTTAAAGCTAAAACAAAAACATGGTTTAATTCATTATCA
GAACAACAAAAAATTGATTTAATACAAGACCTATCAACTATAAATGATTTAACAG
GTTTTGCTCTTAAAAAGAGAATTTATAATCATTGGACAAAAGATGAAGAAATTAT
TGAAAATCTATTATTAACTTCTGATAAACTACCACAAGGTTATACTTCCTTATCG
CTTAAAGCGACAAAAAAAATATTACCATTTTTAAAAGCAGGTAATAAATACTATG
AAGCAGAGAAAAAAGCATACCCTAACTTCACAATTAATACTAAGAAAGTTAATAA
ACTTCCACCAGTTGAGGGTATAAGCAATACAGTTGTATTAAAATCTTGCTCACAA
GTAAGACACCTTGTTAATGCTATTGTTGATAAATATGGTGAAATTGATATTATTA
AAATTGAATTAGCAAGAGATTTATCAAAAAGTAAAAAACAAATTTCTGAAACTAA

TATTTTAAATAGCAAAAATAAATCATTAAATGATGAAGCTATCTCATATTTACATA
AAAATAATATTGACGTTAATCATGACAACATTACTAAATACAAACTATGGAAAGA
AAGCGAAAGTAGTGATAAACAACATCGTTCTTGTTATCCAGAACAAGATAGTGA
TGGTAACTGGTTCTTTCCTACTATTAATTTAAATGACTTGTATGGTGCTAACGCC
ATTTATGAAATAGAACATATTATTCCTAAATCAATATCAGGTGATGATTCATTTGC
AAACAAAGCTTTATGTAAACATAAAACAAATGCTCAAAAAGGTAATAAAACACCT
TATGATTACTATTTTCAAAAAGGTGGTCAAGAACTTATAGATATGATAGCAAAAA
CAGCTTATTCTCAATTTGGTAAATGGAAAGGTAAAAAGTTTGTAACATCTACTGA
AAAATATTTAGATGAAAATGATATTCAAAAACGATACTTAAATGATACTCGCTATT
TAGCTGTATTTTTAAAAGAATATCTTCAACCTGTTTGTAAAGATAAAATTGTTGTT
TCAAAAGGTGGATTTACCGCTATTATTAGACAATCTTTAAATCTTAATATTTTATT
AGGCAATGAATTTAATAAAAATCGTGGTGATTATAGACATCATATGGTTGATGCT
TTTTGTACAAGTCTAATTTCTCCAAAAGTTTTAGATATTATTAATATTATGAGAAA
ACAATTTAATAAGAAAACTGAAAAATATAAAGAGTGTGAAACTAAATTAAATGAT
TATATTAAAAATGTTAAAAATGATTTTATTAAACACTTTGATAATGTTATTACTTCT
CATGAAGTTGAAAGCAAATTAAAAGGTGAATTAGAAGAAACTCTATATGGT
CATGAAAAAATTAAATTAGACGATGGATCTATTATTACAAAACTTTTTCAACATAA
ACCTTATGAAGAAGTTATTTCAAAATACAATAGTCCAGAAGAAGTACTAAAAGCT
CATGATAATTTAGAAATTAGTTTACCAAAAGGTCTAAAAAAATATTTAGAAAACA
ATAATAAACTACCTGATATGTTTCAAGGGTGGAAGCGTATAAAAAAATATTATAA
CAATATTGATGAAAATAATTTTAATATTAATATATATAGAGCTATTCATAATGAAA
AAGAAAAAGTTATTGCTTATAAAAAAACGCAGGAAAATGCTTACATTATTGTTTA
CAATGATTTTAAAAATGAAACCATTAGTAAAATGAATGAAATATCTAATAAACCTA
AATTTAATAATGTATTTTGCAAATTATATAAAGGAGATTTAATCCAAGATTCTGAA
AATAATATATATAAAATTTATCAACTTTTAAGTGATAAAAGAATAGCTTTACATCC
TGTTAATATTCAACCTATAAAAACTAAAAGAAAATCTGTAAAATTAATTTTTGATA
AAGAAAAAATGTATAAAATAAAAGTAAATATTTTAGGTCATATTATT

As the skilled person understand, if the polynucleotide of the invention has to be introduced in a mammal cell, specifically, in a human cell, the polynucleotide of the invention may comprise a nucleotide sequence encoding to the nuclease of the invention, wherein said nucleotide sequence has been humanized to be optimally expressed in the human cell. Thus, in another particular embodiment of the polynucleotide of the invention, alone or in combination with each and every one of the previous particular embodiments, the nucleotide sequence is humanized, preferably, the humanized sequence encoding the nuclease of the invention is the nucleotide sequence SEQ ID NO: 15.

SEQ ID NO: 15
ACCAAGAAAGTGCTGAGCATCGACCCTGGCGTGACCAGCATCGGCTGG
ACCCTGACCGAGCAGAACGAGAAGGGCCATCTCACAAAGTTTATCGAC
GGCGGCAGCAGAATCTTCAACTCCGTGAAGGAAGCCAAGTCTGGTACA
TTTAAGAACGAGAAACGGCGGGCAAAGCGAACAACAAGAAGGAACAAG
TACAGAAGCAATAGAAGAATGGCCAAACTGGAAAACTACCTGATCAAGA
ACGATTTTCTGTCTAACAAGATCTCCAGCCTGTCAGACCACACAGGCCT
GGTCCAGCAGATCATTGGAAATCCTTATCACCTGAGAAAGAAAGCTCTG
TACGAGCCTCTGACCAACGAGGAATTGGCCTGGGCCATCATCCACCTG
GCTAAAAGAAGAGGCTTCCTGAGCAGCTCTAAAAGCCTGACCCCAGAA
GAAAAGAAAGAACAGGCCAGAGCAGATGAGCTGGCCACCCTCATGAAG
AAAGAGAAGTGCAAGACCCTGGGAGAATACTTCAGCAATCTGTATGATA
AGGGCGAAAAGATCAGAGGCGAAAAGCTGAAAAGACAGCTGATCCAGG
ACGAGCTGGAGCTGATCCTGAACAAGCAGATGGAAACCAACAGCCTGA
TCACCTACAAGTTCGTGAACGATATCAACACCATTATCTTCAACCAGAGA
CCTCTGAAAACCCAGAAAAGAATCTCTCTGTGTAAATTCGAGTCTTTTGG
CAAGATCAAGAAACACACCGCCGCCAAGCTGCATCCTCTGAGCCAGCA
ATTTATACTCTGGGGCTTCATCAACAACCTGAAGTACAATGACAACAGC
GGCGGCATGCAGGAGCTGACCCTGGAACAAAAGAACGTGGTGTTCGAC
CTGCTGTGGACGAACAAGGAAGTGACCTTTAAGAAGATTATCAAAGAGC
TGAAGCTGACCCCTGAGACCACCTTCAACTTTCAGAAGGGCGAGGACG
ACAAGGTGAAGGGCAACCAGCTGCTGAACTACTTCAAGGCCAAGACCA
AGACATGGTTCAACAGCCTGTCTGAGCAGCAGAAGATCGACCTCATCCA
GGACCTGTCTACCATCAATGATCTAACCGGCTTCGCCCTGAAGAAGCG
GATCTACAACCACTGGACCAAGGACGAGGAAATCATTGAGAATCTGCTG
CTGACATCCGACAAACTGCCTCAGGGCTACACCAGCCTGTCGCTGAAG
GCCACAAAGAAGATCCTGCCCTTCCTGAAGGCCGGCAACAAATACTAC
GAGGCCGAGAAGAAGGCCTACCCCAACTTCACCATCAACACGAAAAAA

GTGAACAAGCTCCCACCTGTGGAAGGCATCAGTAATACCGTGGTGCTG
AAGTCCTGTAGCCAAGTGCGGCACCTGGTGAACGCCATCGTTGACAAG
TATGGCGAGATCGACATCATCAAGATCGAGCTGGCCCGGGATCTCTCC
AAGAGCAAGAAGCAGATCTCCGAAACCAACATCCTGAACTCTAAGAATA
AAAGCCTGAATGACGAAGCTATCTCATATCTGCATAAGAACAACATCGAT
GTGAATCACGATAATATCACCAAATACAAACTGTGGAAGGAATCTGAATC
CAGCGATAAACAGCACAGATCTTGTTACCCTGAGCAAGATAGCGACGG
CAATTGGTTCTTCCCTACAATCAACCTGAATGACCTGTACGGCGCCAAC
GCTATCTACGAGATCGAGCACATCATCCCTAAGAGCATCAGCGGCGAC
GACAGCTTCGCCAATAAGGCCCTGTGCAAACACAAAACCAACGCCCAG
AAAGGCAACAAAACACCCTACGACTACTTCCAAAAAGGAGGACAGG
AGCTGATTGACATGATCGCGAAGACAGCCTACAGCCAGTTCGGCAAGT
GGAAGGGAAAGAAGTTCGTGACAAGCACCGAGAAGTACCTGGACGAGA
ACGACATCCAAAAGAGATACCTGAACGACACCAGATACCTGGCCGTGTT
CCTGAAGGAGTACCTGCAGCCCGTGTGCAAGGACAAAATCGTGGTCAG
CAAGGGCGGATTCACCGCCATCATTCGGCAGAGCCTTAACCTGAACAT
CCTGCTGGGAAATGAGTTCAACAAGAACCGGGAGATTACAGACACCA
CATGGTGGACGCTTTTTGCACCTCTCTGATCAGCCCCAAGGTTCTGGAC
ATCATTAACATCATGCGGAAGCAGTTCAACAAGAAAACAGAGAAGTACA
AGGAGTGCGAGACAAAGTTAAACGACTACATCAAGAACGTTAAGAATGA
TTTCATCAAACACTTCGACAATGTGATCACATCCCACGAGGTGGAAAGC
AAACTGAAGGGCGAGCTGGAAGAAGACACTGTACGGCCACGAGAAG
ATCAAGCTGGACGATGGCAGCATCATAACCAAGCTGTTCCAGCACAAGC
CTTACGAAGAGGTGATTAGCAAGTATAACAGCCCTGAGGAAGTGCTGAA
GGCCCACGACAACCTGGAAATCAGCCTGCCAAAGGGCCTGAAAAAGTA
CCTGGAAAACAACAACAAACTGCCCGACATGTTCCAGGGCTGGAAACG
CATCAAGAAGTACTACAACAACATCGATGAGAACAACTTCAACATCAACA
TCTATAGAGCCATCCACAACGAAAAGGAGAAGGTGATCGCCTACAAGAA
GACACAGGAGAATGCTTACATCATCGTGTACAACGATTTCAAGAACGAG
ACGATCAGCAAGATGAACGAAATCAGCAACAAACCTAAGTTCAACAACG
TGTTCTGCAAGCTGTACAAGGGCGACCTGATCCAGGATTCTGAGAACAA
CATCTACAAGATATACCAGCTGCTGAGCGACAAGAGAATCGCCCTGCAC

CCCGTGAACATCCAGCCTATCAAAACCAAGCGCAAGAGCGTGAAACTG
ATCTTTGATAAGGAGAAGATGTACAAGATCAAGGTGAACATCCTGGGTC
ACATCATC

### Vector of the invention

A polynucleotide comprising a nucleotide sequence encoding a nuclease of the invention, or the guide RNA (dual or single) of the invention, may be introduced into a cell.

Thus, in another aspect, the present invention relates to a vector, hereinafter "vector of the invention", comprising:
- the polynucleotide of the invention, and/or
- the dual or single guide RNA of the invention.

As used herein, the term "vector" or "plasmid" refers to any vehicle used to transfer foreign genetic material into a cell. Said vector can be a viral vector or a non-viral vector, such as a plasmid. By way of a non-limiting illustration, said vectors can be viral vectors based on retroviruses, adenoviruses, alphaviruses, etc., or in case of non-viral vectors, the vectors can be DNA-liposome, DNA-polymer, DNA-polymer-liposome complexes, pSilencer 4.1-CMV (Ambion), pcDNA3, pcDNA3.1/hyg pHCMV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL and pKSV-10, pBPV-1, pML2d, pTDTI, etc. Said viral and non-viral vectors can be administered to the cell by the conventional methods widely known in the state of the art.

In a particular embodiment of the vector of the invention, the vector is a plasmid, a retroviral vector, a lentiviral vector, an adenoviral vector, and an adeno-associated viral vector.

The vector may contain, among others, multiple cloning sites, expression regulating sequences, suitable replication origins for the host cell in which the vector is introduced, selection markers, etc. In a particular embodiment, the vector is an expression vector that allows the expression of the nucleotide sequence or polynucleotide in the target cell and generally has a promoter sequence (or expression regulating sequences) that drives the expression thereof. The promoter sequence preceding the polynucleotide is operatively bound to the said polynucleotide. As used in this description, the expression "operatively bound" means that the polynucleotide is within the correct reading frame for its expression under the control of said regulating sequences. The regulating sequences useful for the present invention can be nuclear promoting sequences or, alternatively, enhancer sequences and/or other regulating sequences increasing the expression of the polynucleotide. The promoter can be constitutive or inducible. If the constant expression of the polynucleotide is desired, then a constitutive promoter is used. Examples of well-known constitutive promoters include the cytomegalovirus (CMV) immediate-early promoter, the Rous sarcoma virus promoter, and the like. A number of other examples of constitutive promoters are well-known in the art and can be used in implementing the invention. If the controlled expression of the polynucleotide is desired, then an inducible promoter must be used. In a non-induced state, the inducible promoter must be "silent". "Silent" is understood to mean that in the absence of an inducer, little or no expression of the polynucleotide is detected; in the presence of an inducer, however, the expression of the polynucleotide occurs. The expression level can frequently be controlled by varying the concentration of the inducer so that the inducible promoter is stimulated more strongly or weakly and consequently, the concentration of the polynucleotide transcribed product is affected. Examples of well-known inducible promoters include, without limited to, an androgen or estrogen-responsive promoter, a doxycycline-responsive promoter, a metallothionein promoter, or a promoter responding to ecdysone. Other various examples are well known in the art and can be used for implementing the invention.

### Cell of the invention

In order to express the polynucleotide of the invention, the dual guide RNA of the invention; and/or the single guide RNA of the invention, they have to be introduced in an expression system such as a host cell. Taking this into account, the nucleotide sequence may be optimized for a proper expression in the chosen host cell. Thus, in another aspect, the present invention relates to a cell, hereinafter "cell of the invention", comprising (or consisting of):
- (i) the ribonucleoprotein of the invention, and/or
- (ii) the nuclease of the invention, and/or
- (iii) the polynucleotide of the invention, and/or
- (iv) the dual or single guide of the invention, and/or
- (v) the vector of the invention.

The term "host cell" means any cell type that is susceptible to transformation, transfection, transduction, and the like with the nucleotide sequences or vector of the invention.

The vector introduced into a host cell may be maintained as a chromosomal integrant or as a self-replicating extra-chromosomal vector. The term "host cell" also encompasses any progeny of a parent cell that is not identical to the parent cell due to mutations that occur during replication.

The host cell may be any cell, e.g., a prokaryote or a eukaryote such as a mammalian, insect, plant, or fungal cell. Any method known in the art for introducing DNA into a host cell can be used in the context of the present invention.

The prokaryotic host cell may be any Gram-positive bacterium or Gram-negative bacterium. Gram-positive bacteria include, but are not limited to, *Bacillus, Streptococcus, Streptomyces, Staphylococcus, Enterococcus, Lactobacillus, Lactococcus, Clostridium, Geobacillus,* and *Oceanobacillus.* Gram negative bacteria include, but are not limited to, *E. coli, Pseudomonas, Salmonella, Campylobacter, Helicobacter, Flavobacterium, Fusobacterium, Neisseria,* and *Ureaplasma.* In a particular embodiment, the host cell is *E. coli.* Examples of *E. coli* strains useful for vector propagation and cloning procedures include, without limiting to, *E. coli* DH10B (Invitrogen), DH5α (ATCC, Invitrogene), DM1 (Invitrogen), GeneHogs (Invitrogen), HB101 (Bio-Rad, Invitrogene, Promega), INV110 (Invitrogen), JM109 (ATCC, Promega), JS5 (Bio-Rad), LE392 (ATCC), NM522 (AS), SCS110 (Stratagene), STBL4 (Invitrogen), SURE (ATCC, Stratagene), TG1 (Stratagene), XL10-Gold (Stratagene) and XL1-Blue MRF (Stratagene).

Examples of insect cells include, without limiting to, *Spodoptera frugiperda* cells (such as SF9 cell or SF21 cell or Sf900+), a BTI-Tn-5B1-4 insect cell from Trichoplusia ni (High-five^{™}, Invitrogen, Carlsbad Calif.), expresSF+^{®}, Drosophila Schneider 2 (S2) Cells, Se301, SelZD2109, SeUCR1, MG-1, Tn368, HzAm1, Ha2302, Hz2E5 and High Five from Invitrogen.

Examples of the fungi host cells include, without limiting to, Aspergillus (e.g. *Aspergillus awamori, Aspergillus fumigatus, Aspergillus foetidus, Aspergillus japonicus, Aspergillus nidulans, Aspergillus niger* or *Aspergillus oryzae* cell), Aureobasidium, Bjerkandera, Ceriporiopsis (e.g. *Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora),* Chrysosporium (e.g. *Chrysosporium keratinophilum, Chrysosporium lucknowense, Chrysosporium tropicum, Chrysosporium merdarium, Chrysosporium inops, Chrysosporium pannicola, Chrysosporium queenslandicum, Chrysosporium zonatum*)*,* Coprinus (e.g. *Coprinus cinereus*)*,* Coriolus (e.g. *Coriolus hirsutus*)*,* Cryptococcus, Filibasidium, Fusarium (e.g. *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell), Humicola (e.g. *Humicola insolens, Humicola lanuginosa*)*,* Magnaporthe, Mucor (e.g. *Mucor miehei*)*,* Myceliophthora (e.g. *Myceliophthora thermophila*)*,* Neocallimastix, Neurospora (e.g. *Neurospora crassa*)*,* Paecilomyces, Penicillium (e.g. *Penicillium purpurogenum*)*,* Phanerochaete (e.g. *Phanerochaete chrysosporium*)*,* Phlebia (e.g. *Phlebia radiata*)*,* Piromyces, Pleurotus (e.g. *Pleurotus eryngii*), Schizophyllum, Talaromyces, Thermoascus, Thielavia (e.g. *Thielavia terrestris*), Tolypocladium, Trametes (e.g. *Trametes villosa or Trametes versicolor*)*,* or Trichoderma (e.g. *Trichoderma harzianum, Trichoderma koningii, Trichoderma longibrachiatum, Trichoderma reesei, or Trichoderma viride* cell) cell.

The host cell may also be a yeast cell. "Yeast" as used herein includes ascosporogenous yeast (Endomycetales), basidiosporogenous yeast, and yeast belonging to the Fungi Imperfecti (Blastomycetes). More preferably, the yeast host cell is a Candida, Hansenula, Kluyveromyces (e.g. *Kluyveromyces lactis*)*,* Pichia (e.g. *Pichia pastoris*)*,* Saccharomyces (e.g. *Saccharomyces carlsbergensis, Saccharomyces cerevisiae, Saccharomyces diastaticus, Saccharomyces douglasii, Saccharomyces kluyveri, Saccharomyces norbensis, or Saccharomyces oviformis,* etc.), Schizosaccharomyces, or Yarrowia cell (*Yarrowia lipolytica*)*.*

The host cell may also be microalgae. Examples of microalgae include, without limiting to, microalgae from the genera *Chlamydomonas* (e.g. *Chlamydomonas reinhardtii*), *Botryococcus, Neochloris, Nannochloropsis, Phorphyridium, Scenedesmus, Chlorella, Tetraselmis* and *Spirulina.* The chloroplast of these algae are particularly well suited to the production of bacterial proteins as it mimics the native bacterial expression environment of these proteins while being devoid of their cell wall target.

The host cell may also be a plant cell, wherein the vacuolar deposition of recombinant proteins in plant vegetative organs is used to increase yields. To achieve this, the nucleotide sequence of the invention may be fused to sequence-specific vacuolar sorting signals (ssVSS) typical of proteases or proteinase inhibitors and/or Ct-VSS representative of storage proteins or plant lectins. Both types of motifs were capable of increasing accumulation. Importantly, the type of VSSs or position, either the N or C-terminus, did not alter protein stability, their levels or post-translational modifications. Examples of plant cells include, without limiting to, *Nicotiana* sp. (e.g. *N. tabacum, N. benthamiana,* etc.) sugarcane, tomato (*Solanum lycopersicum*) and carrot (*Daucus carota*)*.*

Examples of mammal cells which can be used in the context of the present invention include, without limiting to, a T-cell, a B-cell, an NK cell, epithelial cell lines (porcine, human, etc.), osteosarcoma cell lines (human, etc.), neuroblastoma cell lines (human, etc.), epithelial carcinomas (human, etc.), glial cells (murine, human, etc.), liver cell lines (monkey, etc.), CHO cells (ChineseHamsterOvary), COS cells, BHK cells, HeLa, 911, AT1080, A549, 293 or PER cells. C6, NTERA-2 human ECC cells, D3 cells of the mESC line, human embryonic stem cells such as HS293 and BGV01, SHEF1, SHEF2 and HS181, NIH3T3, 293T, REH and MCF-7 cells and hMSCs (human mesenchymal stem cells), and GliNS2 cells (glioma stem cells). In particular embodiment of the cell of the invention, the cell is mammal cell.

### Uses of the invention

The ribonucleoprotein complex, the dual or single guide RNA, and nuclease described herein are capable of recognizing, binding to, and optionally nicking, unwinding, or cleaving all or part of a target sequence.

Another aspect of the invention refers to the use of (i) the ribonucleoprotein complex of the invention, and/or (ii) the dual or single guide RNA of the invention, and/or (iii) the vector of the invention, and/or (iv) the nuclease of the invention, and/or (v) the polynucleotide of the invention, for modifying *in vitro* target nucleic acid sequence, that comprises binding, cutting or cleaving specifically a target nucleic acid sequence, wherein the elements (i) to (v) are not used for modifying the germ line genetic identity of human beings.

Another aspect of the invention refers to (i) the ribonucleoprotein complex of the invention, and/or (ii) the dual or single guide RNA of the invention, and/or (iii) the vector of the invention, and/or (iv) the nuclease of the invention, and/or (v) the polynucleotide of the invention, for use in therapeutic modifying a target nucleic acid sequence that comprises binding, cutting or cleaving specifically a target nucleic acid sequence.

Alternative to the uses of the invention, the present invention also encompasses a method for modifying *in vitro* a target nucleic acid sequence, comprising putting into contact the (i) ribonucleoprotein complex of the invention, and/or (ii) the dual or single guide RNA of the invention, and/or (iii) the vector of the invention, and/or (iv) the nuclease of the invention, and/or (v) the polynucleotide of the invention, with a target nucleic acid sequence, wherein the method is not a process for modifying the germ line genetic identity of human beings.

In addition, the present invention also encompasses a method for modifying a target nucleic acid sequence modification in a non-human eukaryotic cell, comprising contacting the non-human eukaryotic cell with (i) the ribonucleoprotein of the invention, and/or (ii) the dual or single guide of the invention, and/or (iii) the vector of the invention, and/or (iv) the nuclease of the invention, and/or (v) the polynucleotide of the invention.

In a particular embodiment of the use of the invention, the cutting or cleavage of a target nucleic acid sequence is carried out in a temperature range from 0°C to 38°C, particularly from 15°C to 35°C, more particularly at 20°C, still more particularly at 4°C. In addition, the nuclease without gRNA rapidly inactivated at temperatures over31°C. In all ranges mentioned in the present description the extremes are included.

The concept to "modify in vitro a target nucleic acid sequence" refer herein to produce an altered target site. An "altered target site", "altered target sequence", "modified target site", "modified target sequence" are used interchangeably herein and refer to a target sequence as disclosed herein that comprises at least one alteration when compared to non-altered target sequence. Such "alterations" include, for example: (i) replacement of at least one nucleotide, (ii) a deletion of at least one nucleotide, (iii) an insertion of at least one nucleotide, (iv) a chemical alteration of at least one nucleotide, or (v) any combination of (i) - (iv). A "modified nucleotide" or "edited nucleotide" refers to a nucleotide sequence of interest that comprises at least one alteration when compared to its non-modified nucleotide sequence. Such "alterations" include, for example: (i) replacement of at least one nucleotide, (ii) a deletion of at least one nucleotide, (iii) an insertion of at least one nucleotide, (iv) a chemical alteration of at least one nucleotide, or (v) any combination of (i) - (iv).

All the definitions and particular embodiment of the uses of the invention are applicable to the method of the invention.

### DESCRIPTION OF THE DRAWINGS

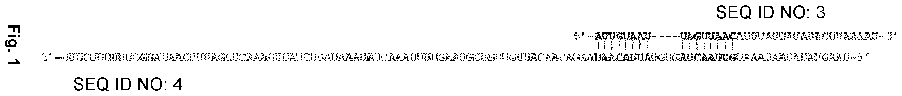

**Figure 1****.** Secondary structure of dual guide RNA of the invention formed by pairing of two sequences, crRNA and tracrRNA, sense vs antisense. The antisense nucleotide sequence of tracrRNA is identified as SEQ ID NO: 4 and the sense nucleotide sequence of crRNA is identified as SEQ ID NO: 3.

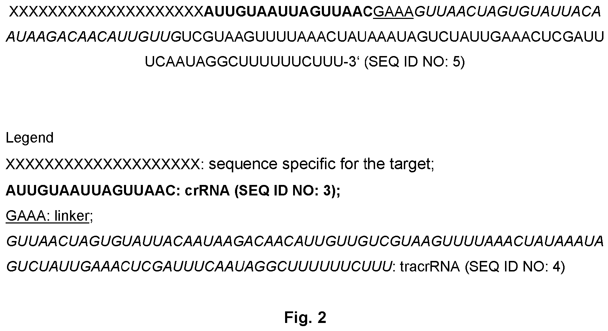

**Figure 2****.** Sequence of single RNA guide of the invention.

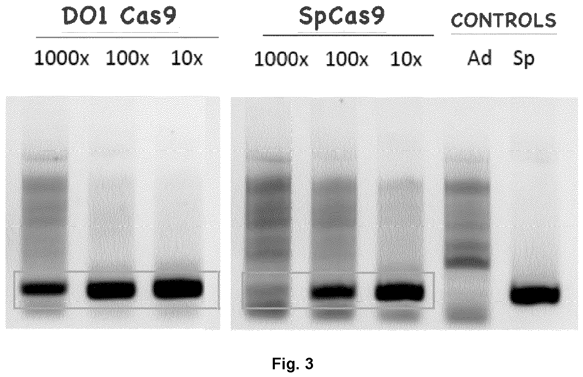

**Figure 3****.** Dilutions of the IVT (in vitro translation) products were tested for cleavage activity on the PAM library by PCR amplification of the product containing the adapter ligated after the DNA cut. Legend: bands indicate activity in cutting plasmids. Controls: Ad, uncut library (negative control); Sp; PCR from protospacers, (positive control).

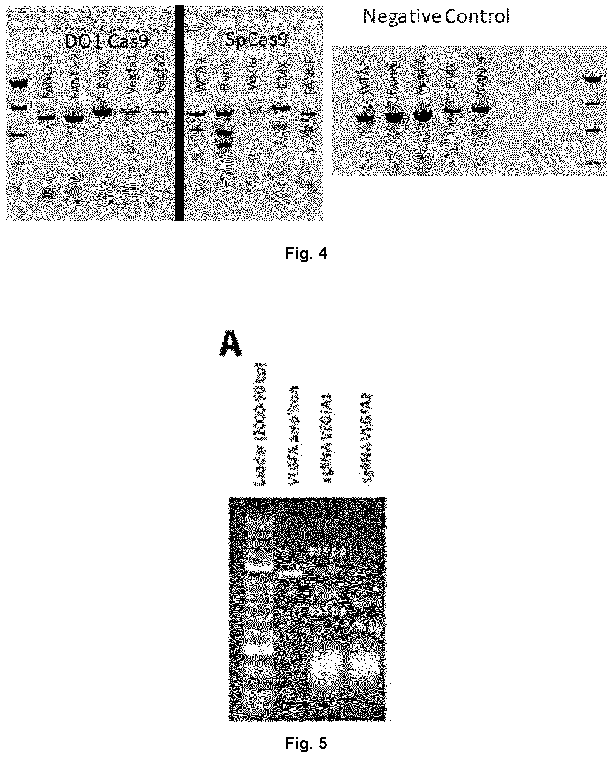

**Figure 4****.** T7 endonuclease I assay to determine nuclease activity of the human codon optimized nuclease of the invention (DO1 Cas9) and SpCas9 RNPs, expressed from plasmids, on human genes in HEK 293T cell line.

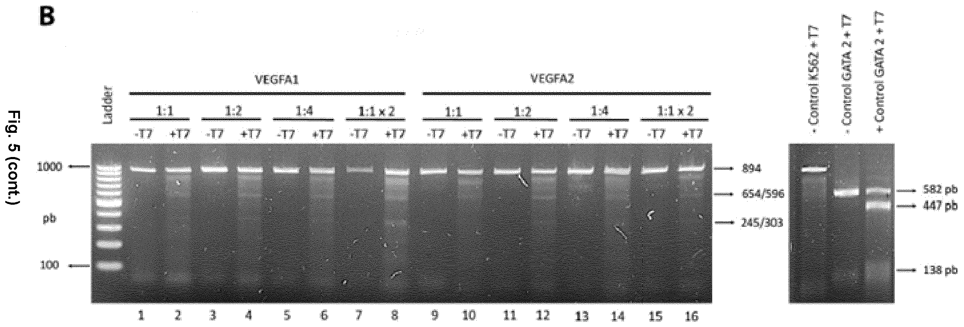

**Figure 5****.** Nuclease activity of nuclease of the invention (DO1 Cas9) and SpCas9 RNPs on human genes. (A) In vitro activity of nuclease of the invention as RNP using two different guide RNAs (VEGFA1 and VEGFA2) for VEGFA gene target sites. 1 µL of purified ID354 protein (32 µM) was mixed with sgRNAs in a 1:1 molar ratio of sgRNA to Cas9 in nuclease-free water and CutSmart buffer (NEB B7200S). The RNP mix was incubated at 25°C for 10 minutes. Subsequently, the substrate was added at a Cas9:sgRNA:DNA ratio of 10:10:1 (100 nmoles of DNA), and the reaction was further incubated at 37°C for 30 minutes. To stop the reaction, 1 µL of Proteinase K (10 mg/µL) was added, and the mixture was incubated at 56°C for 15 minutes (B) Left, T7 endonuclease I assay to determine in vivo nuclease activity of nuclease of the invention (DO1 Cas9) in K562 human malignant melanoma cells. VEGFA gene was targeted at different ratios protein:gRNA; Right, T7 endonuclease I assay to determine in vivo nuclease activity of SpCas9 in K562 cells on GATA2 gene.

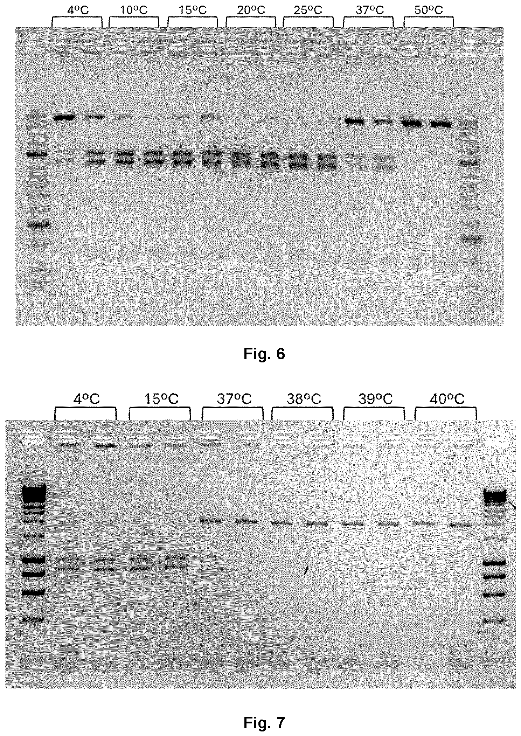

**Figure 6****.** In vitro nuclease activity of RNP of the invention in a temperature range from 4°C to 50°C. In vitro cut of a 1833bp *dpy-10* gene fragment using DO1 Cas9 RNP. Three bands indicate nuclease cut. Single band indicates no activity. Note the decrease of activity of nuclease of invention RNP at 37°C. Reaction mix: DO1 Cas9 (5.1uM) 2.5µl; gRNA (8µM) 1.5µl, H2O 21 µl, 3.1 Buffer 3µl, DNA template 2µl. RNP formation was induced by incubating DO1 Cas9 and gRNA for 30 minutes at 20°C.
Then, a 5-minute preincubation of RNP and the template DNA was performed in separate tubes at the same temperature as the following digestion. Fragments were digested for 30 minutes at the indicated temperatures. Digestions were performed in duplicate for each temperature.
**Figure 7****.** In vitro nuclease activity of RNP of the invention in a temperature range from 4°C to 40°C. RNP activity of nuclease of invention goes down at 37°C and disappears at 39°C. Reaction at similar conditions than Figure 6. For each temperature, a duplicate of the reaction was performed.

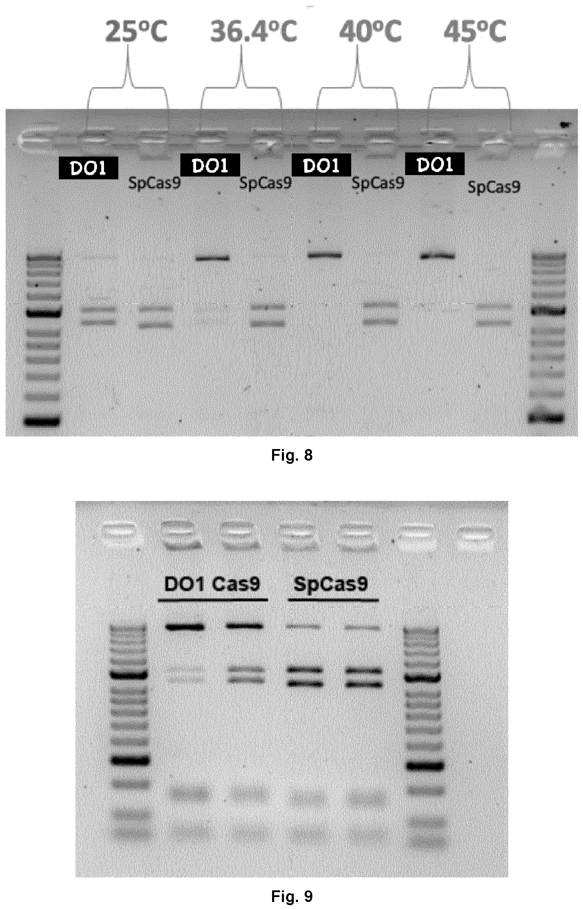

**Figure 8****.** RNP thermostability of nuclease of invention (DO1 Cas9) compared to SpCas9. Once the RNP was complexed at 20°C, a challenging incubation at different temperatures was applied to the preformed RNP prior to the DNA digestion. Differently from SpCas9, the RNP of nuclease of the invention loses its activity beyond 36.4°C. Conditions: RNP formation at 20°C for 30 minutes. RNP complexes challenged at different temperatures and frozen at -20°C. Two hours later, RNP complexes were incubated for 5 minutes at 25°C. Addition of DNA template. Digestion at 25°C for 30 minutes and inactivation.
**Figure 9****.** RNP in vitro activity of nuclease of invention and SpCas9 when the RNP formation and DNA digestion are performed at 4°C.

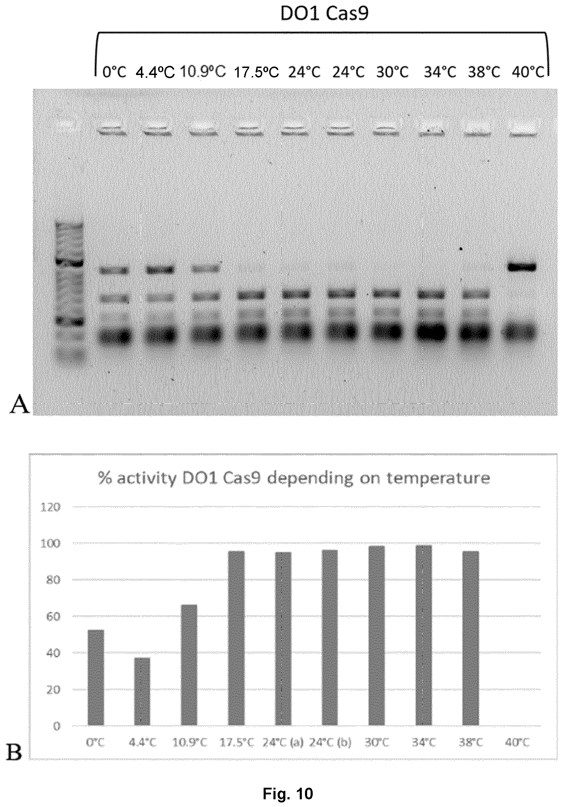

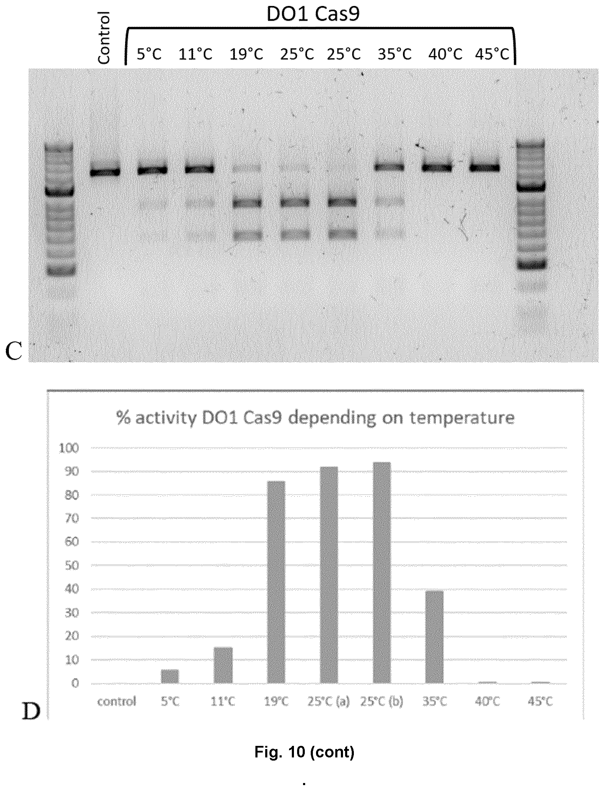

**Figure 10****.** Nuclease of the invention in vitro activity between 0°C and 40°C. (A) agarose gel with digested and undigested products with nuclease of the invention at protein concentration 0.5µM. (B) Quantification of digested bands by densitometry with Image J, normalized to 40°C as reference for lack of activity. Point at 24°C is duplicated because two separate incubation steps in same thermocycler were used to cover the complete temperature range. (C) Agarose gel with digested and undigested products in an independent experiment with DO1 Cas9 at protein concentration 0.35µM. (D) Quantification of digested bands by densitometry with Image J, normalized to control (only template DNA) as reference for lack of activity. Point at 25°C is duplicated because two separate incubation steps in same thermocycler were used to cover the complete temperature range

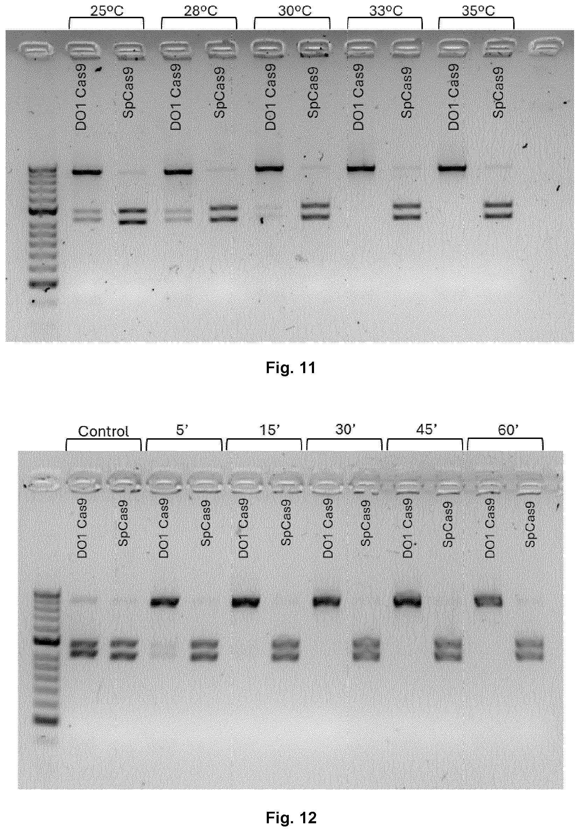

**Figure 11****.** Protein thermostability of nuclease of invention (DO1 Cas9) compared to SpCas9 at a temperature range between 25°C and 35°C. Before the formation of the RNP, an incubation of 1hour at different temperatures was applied to the protein. Then, the RNP was complexed at 20°C for 30 minutes and DNA was later added for digestion at 25°C for 30 minutes. Note that DO1 Cas9 RNP nuclease activity is inactivated when the protein has been challenged at 33°C.
**Figure 12****.** Protein thermostability of nuclease of invention (DO1 Cas9) compared to SpCas9 when incubated at 33°C for different periods of time. RNP in vitro digestion after incubation of DO1 Cas9 or SpCas9 nucleases at 33°C for 5, 15, 30, 45 and 60 minutes. In control condition, proteins were not challenged, the RNP was directly formed at 20°C for 30 minutes. Although complete inactivation of DO1 Cas9 activity is reached after 15 minutes incubation at 33°C, the nuclease activity is already negatively affected after only 5 minutes of incubation.

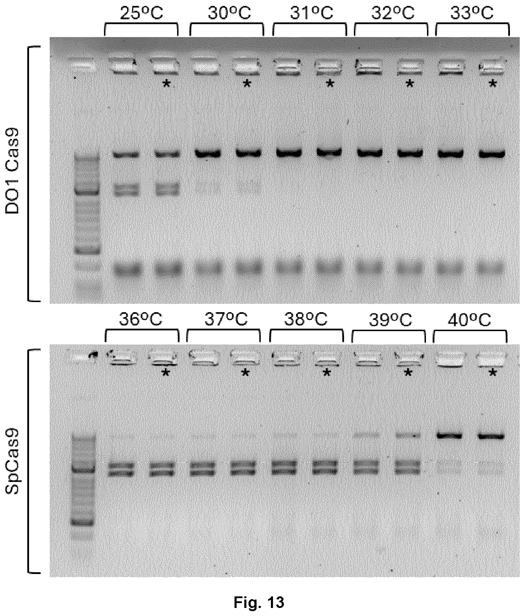

**Figure 13****.** To know with better precision the inactivation temperature of the nuclease of the invention (DO1 Cas9), the protein was heated at 25°C (control), 30°C, 31°C, 32°C and 33°C for 1 hour before forming the RNP. The DO1 Cas9 nuclease activity is inactivated at 31°C and its activity is negatively affected at 30°C. SpCas9 still presents nuclease activity after being pre-heated for 1 hour at 39°C. RNP formation: 20°C 30min. Digestion: 25°C 30min. Lanes marked with (*) correspond to reactions that were frozen after the challenging temperatures and before the formation of the RNP. Note that the inactivation of the nuclease activity is irreversible even after freezing the protein.

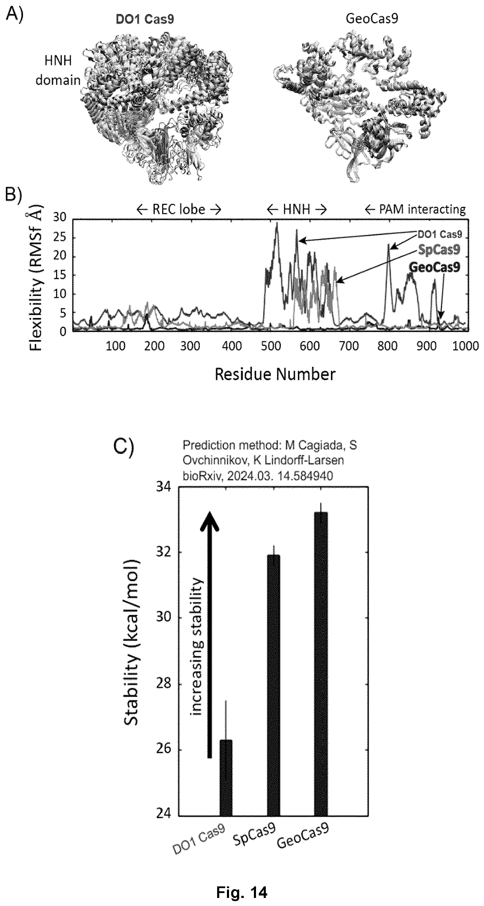

**Figure 14****.** Structural characterization of nuclease of the invention (DO1 Cas9) by computational models. (A) The 3D AlphaFold2 models show typical Cas9 domains, but with extraordinary structural heterogeneity. The HNH nuclease domain is shown in ribbons and its catalytic histidine in spheres. (B) Room Mean Square fluctuation (RMSf) of five DO1 Cas9 models as a function of its sequence compared to those of SpCas9, and a thermostable Cas9 protein of similar length modeled with AF2. (C) Protein folding stability predicted for the structures of DO1 Cas9 (AF2, 5 models), SpCas9 (AF2, 1 model), and GeoCas9 (AF2, 5 models).

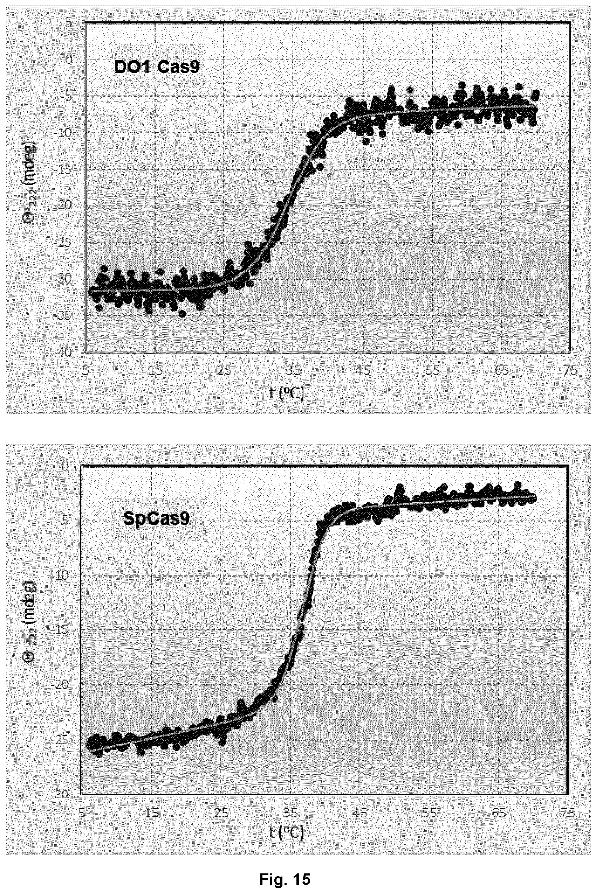

**Figure 15****.** Circular dichroism measurements showing temperature dependence of the ellipticity at 222 nm for nuclease of the invention (DO1 Cas9) and SpCas9.

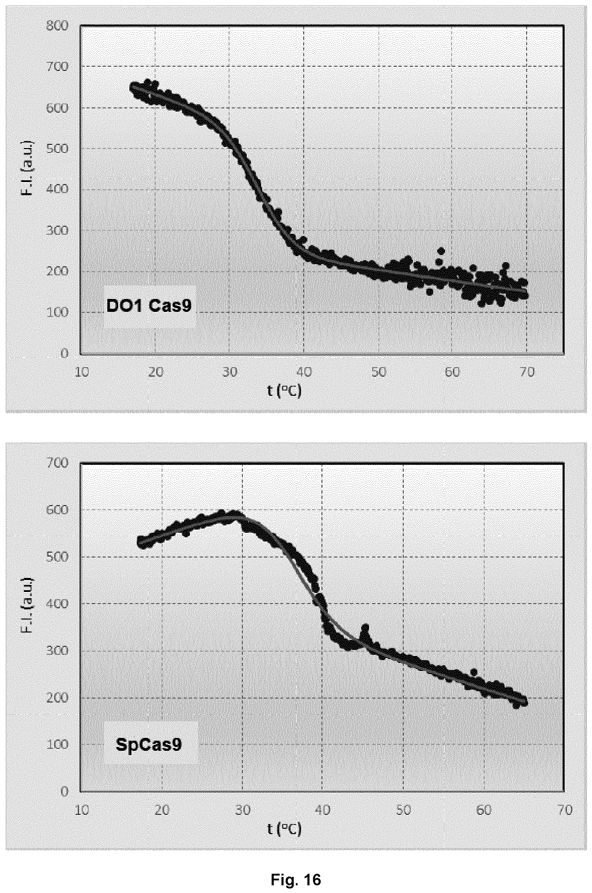

**Figure 16**. Fluorescence spectroscopy measurements showing temperature dependence of fluorescence intensity at 330 nm for nuclease of the invention (DO1 Cas9) and SpCas9.

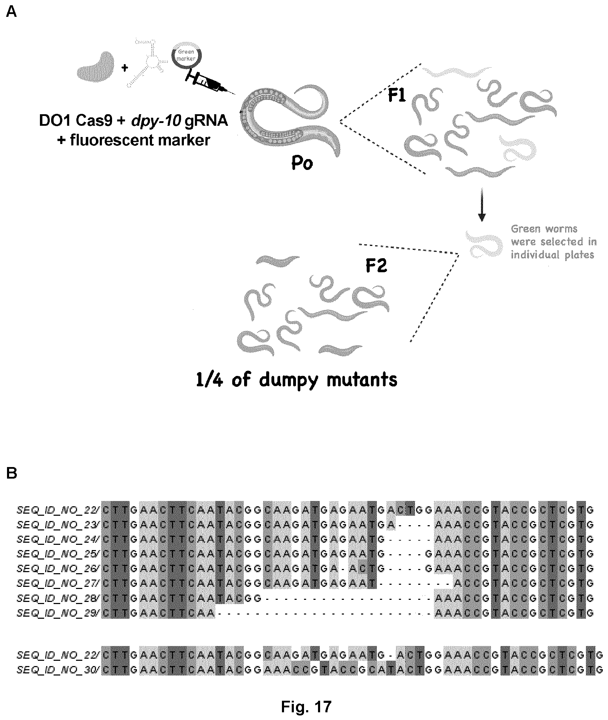

**Figure 17.** (A) Scheme of nuclease of the invention (DO1 Cas9) editing in *C*. *elegans.* 33 worms were edited, evidenced by acquiring the dumpy phenotype, with DO1 Cas9 among 91 well-injected animals (Green fluorescence as marker for a good microinjection in F1), which implies 36% of efficiency for DO1 Cas9 in genome editing in animals. (B) Representative sequences, obtained by Sanger sequencing, of the mutated *dpy-10* targeted region by DO1 Cas9 in animals displaying the dumpy phenotype.

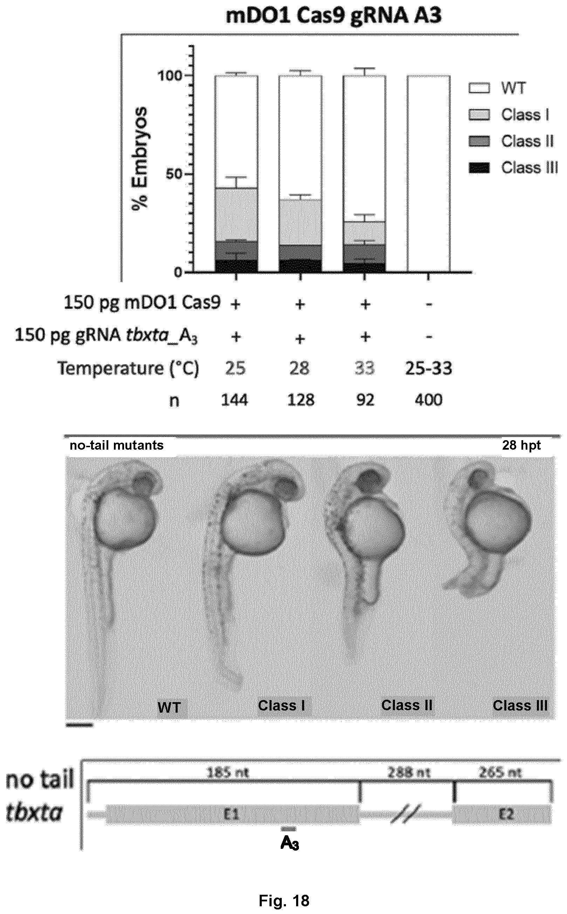

**Figure 18****.** Top panel, percentage of embryos edited on tbxta gene with nuclease of the invention (DO1 Cas9) (injected as mRNA) at different temperatures. Light and dark grey, and black, indicate editing in Zebrafish embryos. Bottom panel, no-tail phenotypes derived of editing tbxta gene and scheme of the tbxta gene and the targeted site (A3).

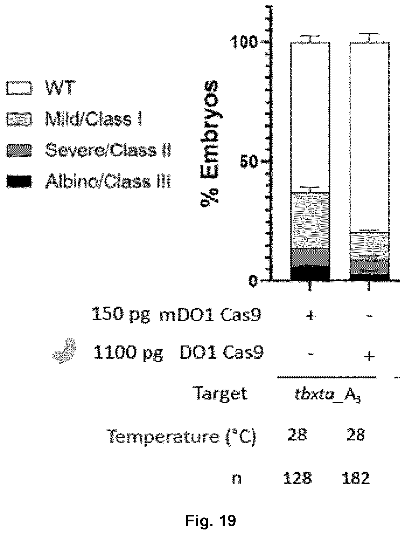

**Figure 19****.** Editing of the tbxta zebrafish gene using mRNA (mDO1 Cas9) or purified protein (DO1 Cas9) to provide nuclease of the invention.

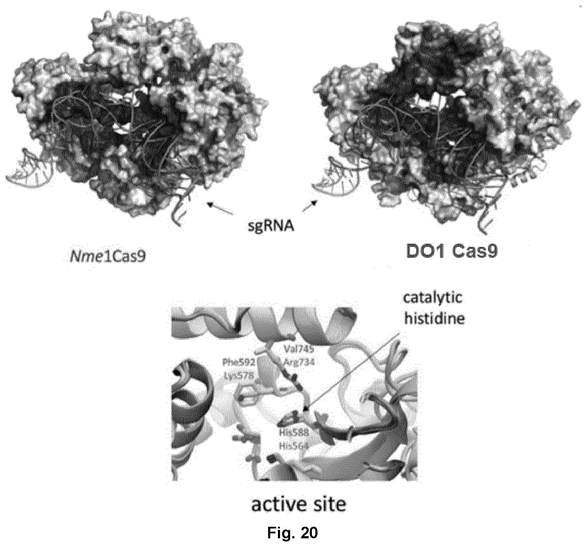

**Figure 20****.** Alpha fold model for the structure of Nme1 Cas9 (another type II-C Cas9) and nuclease of the invention (DO1 Cas9) pointing to a key histidine at the catalytic site.

### EXAMPLES

Next, the invention will be illustrated through tests carried out by the inventors that reveal the psychrophilic nature of the nuclease of invention and its capacity to edit DNA sequences.

### Example 1. Sample collection and identification of nuclease

### 1.1 MATERIALS AND METHODS

### 1.1.1 Sample collection and generation of the Malaspina Vertical Profiles Gene Database

Cas9 sequence derives from the sequencing of metagenomes from the Malaspina Expedition 2010, a circumnavigation of the research vessel Hesperides that collected samples in from 33 sea stations, including 13 vertical profiles with 7 depths from surface to 4000 m. Specifically, Cas9 was identified in a sample collected at 1500 meters deep in the Atlantic Ocean (station 135). Extracted DNA was sequenced on the Illumina HiSeq 2000 platform, in a paired-end mode (2x101bp). Metagenomic from Fastq files analysis was carried out with the following pipeline: sequencing reads were assembled with MegaHit v1.1.3, prokaryotic contigs (contiguous fragments of DNA sequence from an incomplete draft genome) were filtered with Tiara v1.0.2, and genes were predicted with Prodigal in Prokka v1.15 on the assembled contigs. Genes were clustered at 95% of sequence similarity with CD-HIT-est v4.6.1 to produce a 17.4 million non-redundant gene catalog and 2,672 metagenome-assembled genomes (MAGs) of Archaea and Bacteria (Malaspina-VP-MAGs).

### 1.1.2 Identification of nuclease

The 17.4 million non-redundant gene catalog metagenomes of the Malaspina Expedition were examined, identifying 528,589 genes that shared any similarity to any of the 437 Cas profiles that formed our custom database. Out of these, only 4,707 genes were considered to belong to a CRISPR loci. In total, 1,146 CRISPR loci were detected from which 70% were classified unambiguously based on the majority rule output, and 43% contained at least one effector protein and therefore, were considered complete loci. All Cas9 proteins detected were retrieved in the 1,146 CRISPR-Cas loci predicted in the Malaspina Gene Catalog. After discarding truncated proteins, a collection of 48 Cas9 proteins was obtained. Seven type-II Cas9 protein sequences, including DO1 Cas9, were chosen for further validation based on their low homology with other Cas9 proteins, the diverse subtypes (II-A, II-B or II-C), and their suitability to identify the trRNAs.

A phylogenetic tree of Cas9 protein was computed with RAxML v8.2.12 with PROTGAMMALG as the substitution model and 100 bootstrap samplings. The tree included sequences and information about all Cas9 proteins found in the detected loci. The pipeline used was based on Fonfara et al., 2014 *(*Fonfara et al., Nucleic Acids Res. 2014 Feb;42(4):2577-90*).* A set of nine Cas9 proteins was used as queries to perform a Position-Specific Iterated (PSI)-BLAST against the UniRef90 database.

### 1.2 RESULTS

The phylogenetic tree classified nuclease of invention (also called hereinafter as DO1 Cas9), as a Class II Type II-C Cas9 of 991 amino acids (SEQ ID NO: 1), which was isolated from uncultured bacteria in the deep ocean.

The nuclease of the invention is smaller (991 amino acids) than canonical SpCas9 (1386 amino acids) (Class II Type II-A), which was isolated from Streptococcus pyogenes. In addition, the nuclease of the invention presents 31.83% of identity with canonical SpCas9.

### Example 2. Identification of crRNA and tracrRNA to form the gRNA

### 2.1 MATERIALS AND METHODS

A total of seven CRISPR-Cas loci were analyzed by:
i) Predicting the CRISPR array and Cas genes with pilerCR (v1.06), CRISPRCasFinder (v1.1.2) and prokka (v1.14.6).
ii) Aligning the CRISPR DNA (CR) to the locus contig with blast (v2.7.1; -blastn-short -dust no) and considering alignments within 500 bp upstream or downstream of the CRISPR-Cas locus as putative antirepeats (AR) for further analyses.
iii) Looking for Rho-independent terminators (RTS, Rho-independent-like Termination Signal) that are close to the putative antirepeats (FindTerm webserver).
iv) Looking for promoters in the vicinity of the putative antirepeat with BPROM webserver.
v) Checking the interaction between the antirepeat and the CRISPR DNA in IntaRNA (v3.3.1).

### 2.2 RESULTS

When all conditions were checked, the sequences read from 5' to 3' from the antirepeat to the RTS with thepoly-U tail are given as putative tracrRNA sequence for each locus. The transcription sense of the CRISPR repeats was inferred from the best CR-AR interactions, assuming this synteny of the tracrRNA. All coordinates are referred to the original contig coordinates in the plus strand.

Such RNA duplex can work as gRNA (dgRNA) by adding at the 5' end 20 nucleotides corresponding to the 20 nucleotides upstream of the PAM at targeted sequence, or can be shortened to form the following guide RNA (gRNA), which was found functional, by adding a 4 nucleotide GAAA linking a shorter crRNA to shorter tracrRNA to make this final gRNA sequence (sgRNA), being the first 20nt target-specific (Figure 2. Thus, sequences for tracrRNA, crRNA and gRNAs are defined in Table 1.

**Table 1. List of oligonucleotides discover in this study. All the sequences are presented, as conventionally, in 5'-3' sense, with the exception of the first sequence corresponding to Cas9 tracrRNA that is presented in 3'-5' sense (antisense).**

| **Name** | **Sequence** | **Base** |
|---|---|---|
| Cas9 tracrRNA | SEQ ID NO: 4 (antisense) | RNA |
| Cas9 crRNA | SEQ ID NO: 3 (sense) | RNA |
| Cas9 dgRNA | Pairing of these two sequences, sense (Cas9 crRNA) vs antisense (Cas9 tracrRNA), gives the RNA structure of the Figure 1 | RNA |
| Cas9 sgRNA | SEQ ID NO: 5, Figure 2 | RNA |

### Example 3. In vitro translation and transcription, and ribonucleoprotein (RNP) activity on plasmids dependent on the PAM sequence.

### 3.1 MATERIALS AND METHODS

### 3.1.1 In vitro translation and transcription, and RNP activity on plasmids.

Nuclease of the invention was produced by in vitro translation (IVT) using an *E. coli* cell-free transcription-translation system. gRNA was synthetized by T7 in vitro Transcription. The nuclease of the invention was assembled with their corresponding sgRNAs to form nuclease-gRNA complexes (Ribonucleoproteins (RNPs)) that were incubated with a library of plasmids with randomized PAMs sequences of 7 nucleotides. Then, a reaction to ligate an adapter was performed and cleaved products with the adapter incorporated were amplified by PCR and sequenced to determine the PAM sequence. Three different dilutions of the IVT (*in vitro* translation) product were tested for cleavage activity of the PAM library by PCR amplification of the product containing the adapter ligates after the DNA cut (Figure 3).

### 3.1.2 Identification of the PAM sequence

To identify the PAM preferences of Cas9 in vitro, a PAM plasmid library was cleaved with RNPs, adapters were ligated to cleaved fragments and Illumina libraries enriched in cleaved products were PCR amplified and sequenced (Karvelis et al. A pipeline for characterization of novel Cas9 orthologs. Methods Enzymol. 2019;616:219-240)*.*

In brief, 1µg of plasmid library in 90µL of reaction (R) buffer was prepared for each RNP concentration to be tested. Using RNPs assembled directly from *E. coli* lysate or IVT reactions, series of dilutions or distinct concentrations were prepared (e.g., 10X, 100X, 1000X) in CA buffer (10mMTris-HCl, pH 7.5 at 37°C, 100mM NaCl, and 1mM DTT). Next, cleavage reactions are initiated by adding 10ul of the Cas9 RNP complex into the reaction tubes containing 1µg of plasmid library and R buffer (total reaction volume 100µL). Reactions are gently mixed and allowed to proceed for 1h at 37°C. The samples after cleavage were stored at 20°C.

### 3.2 RESULTS

Upon in vitro translation (NT), DO1 Cas9 showed high cleavage activity on plasmids with specific PAM sequences (Figure 3).

Junctions generated by Cas9 cleavage and adapter ligation at different positions of the protospacer (targeted region) were analyzed. PAM preferences at different dilutions were then determined as described in Karvelis et al. 2015 (Karvelis et al. Rapid characterization of CR/SPR-Cas9 protospacer adjacent motif sequence elements. Genome Biol. 2015 Nov 19;16:253) and represented as Position Frequency Matrices (PFMs) (Table 2 and 3) and cut patterns (Table 4). In target strand, most of the cleavage events (>95%) occur in the 3nt upstream of the PAM). Determined Cas9 PAM was: NRVDHCN wherein N is any base, R is A or G, V is A or C or G, D is G or T or A, H is C or T or A, C is Cytosine, and N is any base.

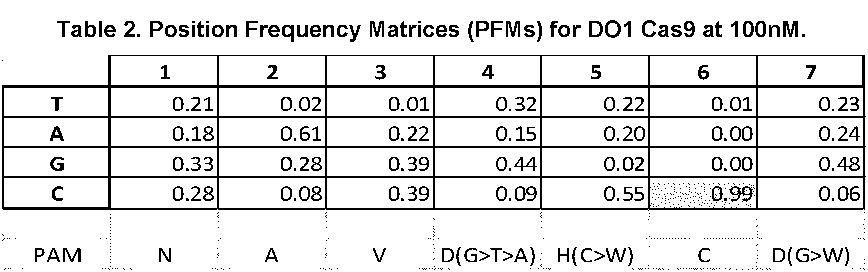

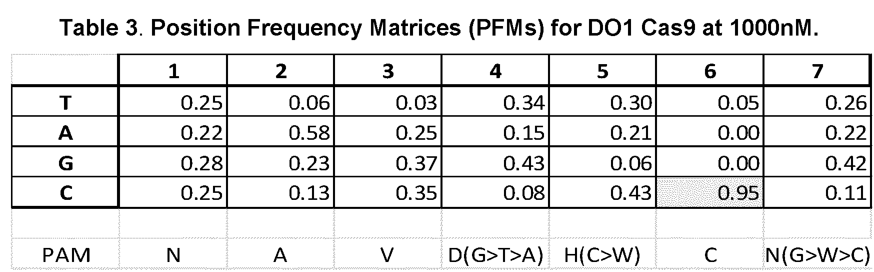

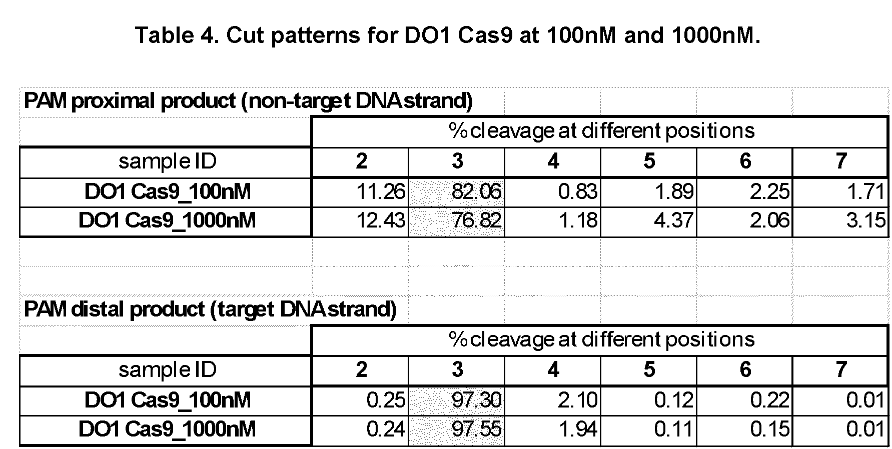

### Example 4. Evaluation of nuclease activity in human cell lines.

### 4.1 MATERIALS AND METHODS

### Use of DO1 Cas9 as plasmid

Sequence of DO1 Cas9 was human codon optimized using genscript algorithms, obtaining the sequence SEQ ID NO: 15. A SV40 Nuclear Location Signal (NLS) was added at the N-terminus and a Nucleoplasmin NLS at C-terminus. The resulting sequence (SEQ ID NO: 18) was cloned into pcDNA3.1(+)-P2A-eGFP vectors over Kpnl and Xbal sites using two flanking sequences SEQ ID NO: 16 and SEQ ID NO: 17. Kpnl [GGTACC], Xbal [TCTAGA] restriction sites were avoided. eGFP is cleaved from the protein by P2A enzyme and allows monitoring the expression level of Cas9. Plasmid and PCR products from sgRNA geneblocks with U6 promoters were transfected into a HEK293T cell line using the FuGene transfection agent (Reagent and DNA ratio 3:1). Cleavage activity in human genes FANCF1m FANCF2m EMX, Vegfa1 and Vegfa2 was analyzed 4 days incubation period after transfection.

### Use of DO1 Cas9 as RNPs

Sequence for DO1 Cas9 codon optimized for *E. coli* expression plus the NLS SV40 and the 6xHis tag (SEQ ID NO: 19) was cloned into the vector pET-21(+) (pET21_DO1 Cas9) for protein purification. The effectiveness of DO1 Cas9 was evaluated via RNP nucleofection targeting the first exon of the VEGFA gene at two different target sites in the K562 human malignant melanoma cell line. sgRNAs were effectively synthesized in vitro from a DNA gBlock using HiScribe^{®}; Quick T7 High Yield RNA Synthesis Kit (NEB #E2050). The synthetized sgRNA product was visualized using SYBR^{™} Gold staining and separated on pre-made Novex TBE-Urea 15% denaturing polyacrylamide gels, yielding satisfactory results in terms of integrity. To assess the functionality of the sgRNAs, they were tested in vitro (Figure 5A), forming RNPs with DO1 Cas9 at 25°C, and followed by incubation with a PCR amplicon of the VEGFA gene derived from the K562 cell line.

To assess whether DO1 Cas9 is capable of editing the VEGFA gene in K652 cells and to determine the most efficient DO1 Cas9:sgRNA ratio, 100 pmoles of DO1 Cas9 were incubated with self-produced guides at different ratios (1:1, 1:2, 1:4), as well as at a 1:1 ratio with double amount (2x) of reagents. This incubation took place at 25°C to form RNPs, which were then used for nucleofection of K562 cells. Editing efficiency was evaluated by a T7 endonuclease assay. This assay employs an endonuclease capable of recognizing heteroduplex regions formed by indels caused by non-homologous end joining (NHEJ) activity.

In the Table 5, the specific spacers (DNA targeted region) for the gRNAs used for targeting each gene are shown.

**Table 5. Specific spacers for the gRNAs used for targeting each gene.**

| **Name** | **Sequence** | **Base** |
|---|---|---|
| FANCF1 | TGCTGACGTAGGTAGTGCTT (SEQ ID NO: 6) | DNA |
| FANCF2 | CCCGGAACTGGACCCCGCCC (SEQ ID NO: 7) | DNA |
| EMX | AGCTGGAGGAGGAAGGGCCT (SEQ ID NO: 8) | DNA |
| VEGFA1 | GAGCCGTGGTCCGCGCGGGG (SEQ ID NO: 9) | DNA |
| VEGFA2 | TGGACGAAAAGTTTCAGTGC (SEQ ID NO: 10) | DNA |

gBlocks and crRNA used for Figure 5:
SEQ ID NO: 11. gBlock for VEGFA sgRNA1 targeting: 
AAGGTCGGGCAGGAAGAGGGCCTATTTCCCATGATTCCTTCATATTTGCATATA
CGATACAAGGCTGTTAGAGAGATAATTAGAATTAATTTGACTGTAAACACAAAG
ATATTAGTACAAAATACGTGACGTAGAAAGTAATAATTTCTTGGGTAGTTTGCAG
 
TTTTAAAATTATGTTTTAAAATGGACTATCATATGCTTACCGTAACTTGAAAGTAT
TTCGATTTCTTGGCTTTATATATCTTGTGGAAAGGACGAAACACCGAGCCGTGG
TCCGCGCGGGGATTGTAATTAGTTAACGAAAGTTAACTAGTGTATTACAATAAG
ACAACATTGTTGTCGTAAGTTTTAAACTATAAATAGTCTATTGAAACTCGATTTCA
ATAGGCTTTTTTCTTT
SEQ ID NO: 12. gBlock for VEGFA sgRNA2 targeting: 
AAGGTCGGGCAGGAAGAGGGCCTATTTCCCATGATTCCTTCATATTTGCATAT
CGATACAAGGCTGTTAGAGAGATAATTAGAATTAATTTGACTGTAAACACAAAG
ATATTAGTACAAAATACGTGACGTAGAAAGTAATAATTTCTTGGGTAGTTTGCAG
TTTTAAAATTATGTTTTAAAATGGACTATCATATGCTTACCGTAACTTGAAAGTAT
TTCGATTTCTTGGCTTTATATATCTTGTGGAAAGGACGAAACACCTGGACGAAA
AGTTTCAGTGCATTGTAATTAGTTAACGAAAGTTAACTAGTGTATTACAATAAGA
CAACATTGTTGTCGTAAGTTTTAAACTATAAATAGTCTATTGAAACTCGATTTCAA
TAGGCTTTTTTCTTTT
SEQ ID NO: 13. DO1 Cas9 crRNA sequence complementary to targeted region of VEGFA 1:
   GAGCCGUGGUCCGCGCGGGG
SEQ ID NO: 14. DO1 Cas9 crRNA sequence complementary to targeted region of crRNA VEGFA2:
   UGGACGAAAAGUUUCAGUGC
SEQ ID NO: 16. 5' Flanking sequence for cloning into a pcDNA3.1(+)-P2A-eGFP vector:
   GGTACCGCCACCATGCCGAAAAAGAAGCGAAAGGTC
SEQ ID NO: 17. 3' Flanking sequence for cloning into a pcDNA3.1(+)-P2A-eGFP vector: 
AAAAGGCCGGCGGCCACGAAAAAGGCCGGCCAGGCAAAAAAGAAAAAGTCTA
GA
SEQ ID NO: 18. DO1 Cas9 codon optimized for expression in human cells with nuclear location signals (NLS):

ATGCCGAAAAAGAAGCGAAAGGTCACCAAAAAAGTGCTGAGCATTGATCCTGG
CGTGACCTCCATCGGATGGACTCTCACAGAGCAGAACGAGAAGGGCCACCTG
ACAAAGTTCATCGATGGCGGAAGCAGAATCTTCAACAGCGTGAAGGAGGCCAA
ATCTGGCACCTTCAAGAATGAGAAGCGGAGAGCCAAAAGAACCACCCGGAGAA
ACAAGTACAGATCTAACAGAAGAATGGCCAAGCTGGAGAACTACCTGATCAAG
AACGATTTCCTCTCTAATAAAATCAGCAGCCTGTCCGACCACACCGGCCTGGT
GCAGCAAATCATCGGCAATCCTTACCACCTGAGAAAGAAAGCCCTTTATGAACC
CCTGACCAATGAAGAGCTGGCATGGGCCATCATCCACCTGGCCAAGAGACGC
GGCTTCCTGAGCTCCAGCAAGAGTCTGACCCCAGAGGAGAAGAAAGAACAGG
CTAGAGCCGACGAGTTGGCTACACTGATGAAGAAGGAAAAGTGCAAGACCCTG
GGAGAGTACTTCAGCAACCTGTACGACAAGGGTGAGAAGATCAGAGGCGAAAA
GCTGAAGCGGCAGCTGATCCAGGACGAGCTGGAACTGATTCTGAATAAGCAAA
TGGAAACAAATAGCCTGATCACCTACAAGTTCGTGAATGATATCAATACCATCA
TCTTCAACCAGAGACCTCTGAAGACCCAGAAAAGGATTAGCCTGTGTAAATTCG
AGTCTTTTGGAAAGATCAAGAAGCACACAGCCGCCAAGCTGCACCCTCTGAGC
CAGCAGTTTATCCTGTGGGGCTTCATTAATAATCTGAAATATAACGACAACAGC
GGCGGCATGCAGGAGCTAACCCTGGAACAGAAAAACGTGGTGTTCGACCTGC
TTTGGACCAACAAGGAAGTGACCTTTAAGAAGATCATCAAAGAACTGAAGCTGA
CCCCTGAAACAACCTTTAACTTCCAGAAAGGCGAAGATGACAAAGTCAAGGGC
AACCAGCTGCTGAACTACTTCAAAGCCAAAACAAAGACCTGGTTCAACAGCCT
GTCTGAACAGCAGAAGATCGATCTGATACAGGACCTGTCTACAATCAACGACC
TGACAGGCTTCGCCCTGAAGAAGCGGATCTATAACCACTGGACCAAGGATGAA
GAGATCATCGAGAACCTGCTGCTGACCTCTGATAAGCTGCCACAGGGCTACAC
CAGCCTTTCTCTGAAGGCCACCAAGAAGATCCTGCCTTTCCTGAAAGCTGGCA
ACAAGTACTACGAGGCCGAGAAGAAGGCCTATCCTAACTTCACCATCAACACC
AAGAAGGTTAACAAATTGCCCCCCGTGGAAGGCATCAGCAACACCGTGGTGCT
GAAGAGCTGCAGCCAGGTGCGGCACCTGGTGAACGCCATTGTGGACAAGTAC

GGCGAGATCGACATCATCAAGATTGAGCTGGCTAGAGACCTGAGCAAAAGCAA
GAAACAAATCTCGGAAACTAATATCCTGAATTCCAAGAACAAGTCCCTGAACGA
CGAGGCTATCAGCTACCTGCACAAGAACAACATCGACGTGAATCACGACAACA
TCACCAAGTACAAGCTGTGGAAGGAAAGCGAATCTAGCGACAAGCAACACAGA
AGCTGCTACCCCGAGCAGGATAGCGACGGCAACTGGTTTTTCCCCACAATCAA
CCTGAATGATCTATATGGCGCCAACGCCATCTACGAGATCGAGCACATCATCC
CTAAGTCCATCAGCGGCGACGATAGCTTCGCCAACAAGGCTCTCTGCAAACAC
AAAACCAATGCCCAGAAGGGAAATAAAACCCCATACGACTACTACTTTCAGAAA
GGAGGACAGGAGCTGATCGATATGATCGCCAAAACAGCCTACAGCCAGTTCG
GAAAATGGAAGGGCAAGAAGTTCGTGACAAGTACCGAGAAATACCTGGACGAA
AATGACATCCAGAAGCGCTACCTCAATGACACCAGATACCTGGCCGTCTTCCT
GAAAGAGTACCTGCAGCCTGTGTGTAAGGACAAGATCGTGGTGTCCAAGGGG
GGTTTTACCGCCATCATTAGACAGAGCCTGAACCTGAACATCCTGCTGGGCAA
CGAATTCAACAAGAATAGAGGCGACTACAGACACCACATGGTCGACGCCTTTT
GCACCAGCCTGATCAGCCCTAAGGTGCTGGACATCATCAACATCATGAGAAAA
CAGTTCAACAAAAAGACAGAAAAGTACAAGGAGTGCGAGACAAAGCTGAACGA
TTACATTAAGAACGTGAAGAACGACTTCATCAAGCATTTCGACAACGTGATCAC
CAGCCACGAGGTGGAATCCAAGCTGAAGGGCGAGCTGGAGGAAGAAACACTG
TACGGCCACGAGAAGATAAAGCTGGATGACGGCAGCATCATCACGAAGCTGTT
CCAACACAAGCCATACGAGGAAGTTATCAGCAAGTACAACTCGCCTGAGGAAG
TCCTGAAGGCCCACGATAACCTGGAGATCAGCCTGCCGAAGGGCCTGAAAAA
GTACCTGGAAAACAACAACAAGCTGCCCGACATGTTCCAGGGCTGGAAACGGA
TCAAGAAGTACTACAACAACATCGACGAGAACAACTTCAACATCAACATCTATC
GGGCGATCCATAACGAGAAAGAGAAGGTGATCGCCTACAAGAAAACACAGGA
GAACGCCTACATCATCGTGTACAACGACTTCAAGAACGAGACAATCTCTAAGAT
GAACGAAATCAGCAACAAGCCTAAGTTTAACAATGTGTTTTGTAAGCTGTACAA
GGGAGATCTGATCCAAGACAGCGAGAACAACATCTACAAGATATACCAGCTGC
TGAGCGACAAGCGTATCGCTCTGCATCCTGTGAACATCCAGCCTATCAAGACC
AAGCGGAAGAGCGTGAAGCTCATCTTCGATAAGGAGAAGATGTACAAGATCAA
GGTGAACATCCTGGGCCACATCATCAAAAGGCCGGCGGCCACGAAAAAGGCC
GGCCAGGCAAAAAAGAAAAAG

### 4.2 RESULTS

### Use of DO1 Cas9 as plasmid at 37°C

Low DO1 Cas9 activity was observed in human cells (HEK 293T cells), particularly in VEGFA targets, compared to SpCas9 (known optimal gRNAf for SpCas9) when DO1 Cas9 was expressed from plasmids and gRNA expressed from a gblock with U6 promoter (Figure 4). Such feature would be used when a milder or lower Cas9 activity is convenient.

### Use of DO1 Cas9 as RNPs

- Modest in vitro cleavage for the VEGFA1 sgRNA guide and complete digestion for the VEGFA2 sgRNA (Figure 5A).
- Nuclease of the invention cleaves the VEGFA gene in K562 cells at the targeted positions 1 and 2 but displays a low or mild activity compared to the control SpCas9 on GATA2 gene (Figure 5B).

### EXAMPLE 5. In vitro nuclease activity

### 5.1 MATERIALS AND METHODS

### Protein purification

Cas9 sequence, *E. coli* optimized plus the NLS SV40 and the 6xHis tag (SEQ ID NO: 19), was cloned into the vector pET- 21(+) (pET21_DO1 Cas9) for expression in *E. coli* (BL21) induced with IPTG. After cell lysate, the protein was purified with affinity columns (IMAC HisHitrap 5ml column, Size Exclusion: SEC200). Purified protein was resuspended in the following buffer: 20 mM Tris-HCl, 500 mM NaCl, 1 mM DTT, 0.1 mM EDTA, 50% glycerol, at pH 7.5.

### Activity in vitro

As a DNA template for testing nuclease activity in vitro, a PCR amplified fragment of 1833 base pairs of the *dpy-10* gene was used. Guides RNA for nuclease of the invention DO1 Cas9 and SpCas9 were synthesized from a Gblock (IDT) of sequences using HiScribe^{®} (SEQ ID NO: 20 y 21, respectively); Quick T7 High Yield RNA Synthesis Kit (NEB #E2050). The common steps of an in vitro reaction experiment include the formation of the RNP by complexing the nuclease with the gRNA (dgRNA or sgRNA) at 20°C for 30 minutes, the DNA digestion of the PCR template at 25°C for 30 minutes, and the inactivation of the reaction by addition of DNA loading dye containing EDTA (BlueJuice^{™}, Invitrogen #10666143), and increasing the temperature to 70°C for 10 minutes. Finally, all reactions were run in a 1,5% agarose electrophoresis gel. Modifications of this protocol include different temperatures and/or duration of the incubations.
SEQ ID NO: 19. DO1 Cas9 codon optimized for *E*. *coli* expression plus the NLS SV40 and the 6xHis tag. 
ATGAAAATAGAAGAGACAAAAAAGGTACTATCGATCGACCCGGGTGTTACCTC
CATCGGCTGGACCTTGACCGAACAAAACGAGAAGGGTCACCTGACGAAGTTCA
TCGACGGCGGCAGCCGTATCTTCAACTCCGTGAAAGAGGCGAAGAGCGGCAC
CTTTAAAAACGAAAAGCGCCGCGCGAAGCGCACCACCCGTCGTAATAAATACC
GCAGCAACCGTCGTATGGCAAAGCTGGAAAACTATCTGATTAAAAACGACTTTT
TATCCAACAAAATCAGCAGCCTGAGCGACCACACGGGACTGGTCCAGCAAATC
ATTGGCAATCCGTATCATCTGCGTAAAAAGGCTCTGTATGAACCGCTGACCAAC
GAAGAGTTGGCCTGGGCAATTATCCACCTGGCGAAGCGCAGAGGCTTCCTATC
AAGCAGCAAGAGCTTGACTCCAGAAGAGAAAAAGGAGCAGGCACGTGCGGAT
GAGTTAGCAACCCTGATGAAAAAAGAGAAGTGCAAAACTCTGGGTGAATACTTC
AGCAACTTGTACGATAAAGGCGAGAAGATCCGCGGTGAAAAGCTGAAACGCCA
GCTGATTCAAGACGAGCTGGAATTGATTCTGAATAAGCAAATGGAAACCAACTC
TCTAATCACCTATAAATTCGTCAATGACATCAACACCATTATTTTTAACCAGCGT
CCGCTGAAGACCCAGAAACGCATCTCTCTGTGTAAATTTGAATCCTTCGGTAAA
ATCAAAAAACACACCGCTGCCAAGCTGCACCCGCTGTCCCAGCAGTTCATTCTT
TGGGGTTTTATCAATAACTTGAAATACAATGACAACTCCGGTGGCATGCAAGAG
CTGACTCTGGAACAAAAAAACGTTGTTTTCGACTTGCTGTGGACGAATAAGGAG
GTGACCTTTAAGAAGATTATCAAGGAGCTGAAGTTGACCCCGGAAACAACCTTC
AACTTCCAAAAGGGCGAAGATGATAAAGTAAAAGGAAACCAGCTGCTGAATTAT
TTCAAAGCGAAGACCAAAACCTGGTTCAACAGTCTGTCCGAGCAACAAAAGATT
GACCTGATCCAGGACCTTAGCACCATTAACGACCTGACGGGTTTCGCCCTAAA
GAAACGCATTTATAACCATTGGACTAAGGATGAAGAGATAATCGAAAATCTCCT
GCTGACCTCAGACAAGCTGCCGCAGGGCTATACCTCTTTGTCTCTGAAGGCGA
 
CCAAGAAAATCCTGCCGTTTCTCAAGGCGGGTAACAAGTATTACGAGGCTGAA
AAAAAGGCGTACCCAAATTTTACCATCAACACTAAAAAAGTTAACAAACTGCCT
CCGGTAGAAGGTATTAGCAATACCGTTGTTCTGAAGAGCTGCAGCCAGGTTCG
TCACCTGGTCAACGCAATTGTTGATAAATACGGCGAAATCGACATCATCAAAAT
TGAGCTCGCGCGTGATTTGTCGAAAAGCAAAAAACAGATTTCTGAGACGAATAT
CCTTAACAGCAAGAACAAAAGCTTGAACGACGAGGCGATTTCCTATCTGCACAA
AAATAACATTGATGTTAATCATGATAACATCACCAAGTACAAGCTGTGGAAAGA
ATCGGAATCTTCTGATAAGCAGCATCGTAGCTGTTATCCGGAGCAAGATTCCGA
CGGTAACTGGTTTTTTCCGACCATCAATCTGAATGATCTGTACGGCGCAAATGC
CATCTATGAAATCGAGCACATTATTCCGAAGAGCATTAGTGGTGATGACTCGTT
CGCAAATAAAGCGCTTTGCAAGCACAAGACGAATGCGCAGAAAGGCAACAAAA
CTCCGTATGATTACTACTTCCAGAAAGGTGGCCAGGAACTGATTGACATGATTG
CGAAGACGGCTTACAGCCAGTTCGGTAAATGGAAGGGCAAAAAGTTCGTGACC
TCCACCGAGAAATACCTGGATGAAAATGACATTCAAAAACGTTACTTGAATGAT
ACCCGTTATCTGGCTGTGTTTCTGAAAGAGTATCTGCAGCCGGTGTGCAAGGA
CAAAATCGTGGTGAGCAAAGGCGGTTTCACCGCAATTATCCGCCAAAGCCTCA
ACCTGAATATACTACTTGGTAACGAATTTAATAAAAATCGTGGTGACTACCGTCA
TCATATGGTTGACGCGTTTTGTACCAGCCTGATTAGCCCGAAAGTGCTGGATAT
CATCAACATTATGCGTAAACAATTTAACAAAAAGACGGAAAAGTATAAGGAGTG
CGAAACCAAGCTCAACGACTACATCAAAAACGTGAAAAACGATTTTATCAAGCA
CTTTGACAACGTTATTACCAGCCATGAGGTCGAAAGCAAGCTGAAAGGTGAATT
GGAAGAGGAGACCCTGTACGGTCACGAAAAGATTAAACTCGACGACGGCTCG
ATAATCACAAAGTTATTTCAGCACAAACCGTACGAGGAGGTTATTAGCAAATAC
AACTCCCCGGAAGAAGTGCTCAAGGCGCATGACAACCTGGAGATCAGCTTGCC
GAAAGGCCTGAAGAAATACTTGGAGAACAATAACAAGCTCCCAGATATGTTTCA
AGGTTGGAAACGGATTAAAAAGTACTACAATAACATCGACGAGAACAATTTCAA
CATCAACATTTACCGTGCGATCCACAACGAGAAAGAGAAGGTGATCGCGTACA
AAAAAACACAAGAAAACGCTTATATTATCGTTTATAACGACTTCAAGAACGAGAC
CATCAGCAAGATGAATGAGATCTCCAACAAACCGAAATTCAACAACGTGTTCTG
CAAGCTGTATAAGGGTGATTTAATACAGGATTCGGAGAACAACATCTACAAAAT
CTATCAGTTGCTGAGCGATAAACGTATAGCGCTGCATCCGGTCAACATTCAACC
GATTAAAACGAAACGTAAATCTGTTAAACTAATCTTCGATAAGGAGAAGATGTAT
AAGATCAAGGTGAACATTCTGGGTCATATTATCTCTCGTGCTGATCCGAAGAAA
AAGAGGAAGGTGGCGGCCGCCCTCGAGCACCACCACCACCACCACTGA
SEQ ID NO: 20. Gblock sequence to synthesize DO1 Cas9 gRNA specific for *dpy-10*: 
5'aagcTAATACGACTCACTATAGGCGGCAAGATGAGAATGACTGATTGTAATTAG
TTAACGAAAGTTAACTAGTGTATTACAATAAGACAACATTGTTGTCGTAAGTTTT
AAACTATAAATAGTCTATTGAAACTCGATTTCAATAGGCTTTTTTCTTT3'
SEQ ID NO: 21. Gblock sequence to synthesize SpCas9 gRNA specific for *dpy-10*:

5’aagcTAATACGACTCACTATAGGCTACCATAGGCACCACGAGGTTTAAGAGCT
ATGCTGGAAACAGCATAGCAAGTTTAAATAAGGCTAGTCCGTTATCAACTTGAA
AAAGTGGCACCGAGTCGGTGCTTTTTTT3’

### 5.2 RESULTS

Nuclease of the invention showed activity in vitro between 0°C and 38°C (Figure 10). Ribonucleoprotein complex of the invention activity drops dramatically at 37°C and disappears at 39°C (Figure 7) whereas SpCas9 RNP still shows good activity at 45°C (Figure 8). At the low temperature range, it was observed that the nuclease of the invention and SpCas9 show good in vitro activity at 4°C after forming RNP at 4°C (Figure 9). Altogether, nuclease of the invention presents good in vitro activity between 0°C and 35°C, being over 10°C and below 37°C the range for optimal activity (Figure 6).

### Example 6. Evaluation of the cold adaptability of nuclease.

### 6.1 MATERIALS AND METHODS

### 6.1.1 Protein stability

In each protein stability experiment involving in vitro digestion of a DNA template, a common mix for all reactions was prepared containing NEB r3.1. buffer, Cas9 protein and nuclease-free H₂O. Then, an equal volume was separated in different PCR tubes. All incubation steps were performed in a thermocycler with a controlled temperature. For the challenging of the Cas9 protein, the above-mentioned reactions were incubated at different temperatures for diverse periods of time. In some cases, the reactions were frozen at -20°C for 2 hours (marked with * in the figures). Then, all reactions were set at 20°C before adding the gRNA. Once the gRNA was included in the reaction, the RNP formation occurred at 20°C for 30 minutes. The DNA template was also incubated in a separate tube for the last 5 minutes in this incubation step. When the RNP was formed, the DNA template was added to the reaction to perform the *in vitro* digestion at 25°C for 30 minutes. After this time, the reaction was stopped by adding a DNA loading buffer containing EDTA (BlueJuice^{™}, Invitrogen #10666143), and increasing the temperature to 70°C for 10 minutes. Finally, all reactions were run in a 1,5% agarose electrophoresis gel.

### 6.1.2 Cold-adapted

Cold-adapted or psychrophilic enzymes present an activity temperature range much lower than their melting point (Nowak and Otzen, BBA Advances, Vo. 5, 2024). Psychrophilic enzymes are cold-active and heat-labile and show improved motility and flexibility at the active sites (Feller & Gerday. Psychrophilic enzymes: hot topics in cold adaptation. Nat Rev Microbiol. 2003 Dec; 1(3):200-8)*.* Besides low thermal stability, cold-adapted enzymes present other biophysics properties such as a low optimal reaction temperature, and a low activation energy for the enzymatic reaction (Liu et al. Advances in cold-adapted enzymes derived from microorganisms. Front Microbiol. 2023 Apr 26;14:1152847).

### Analysis of nuclease structure

In order to evaluate the motility and structural flexibility of the nuclease of the invention, it was structurally characterized using computational models.

*Determination of thermal stability, melting point, and activation energy of nuclease using circular dichroism (CD) and fluorescence spectroscopies (FS).*

Circular dichroism monitors the ellipticity of the protein when the sample is heated at a constant rate (1°C per minute). The physical observable is related to the amount of secondary structure of the protein. At low temperatures, the protein being in its folded state, has a large negative ellipticity indicative of a well-packed conformation in which the different elements of secondary structure interact thermodynamically favourably stabilizing the native (fully active) conformation. The increase in temperature would induce the (partial) unfolding of the protein and, therefore, the loss of some elements of its secondary structure.

The unfolding process is only complete for small globular proteins in very defined conditions (acidic pH, low salt concentration, etc.). For most proteins, the initial unfolding of the protein leads to the population of partially folded conformations with the simultaneous exposure to the solvent of large patches of apolar groups (previously buried in the native conformation). As a result, these partially folded states tend to agglomerate together to avoid the hydration of the exposed hydrophobic patches leading to the precipitation of the denatured protein.

The denaturation of the protein is therefore under kinetic control since the reverse reaction (dissolution of the aggregated protein and its concomitant folding to reach the initial native state) has a negligible rate. In fact, the final state is kinetically trapped since the energy barriers needed to overcome the aggregated state needed to reach a soluble one are huge and therefore unattainable within a reasonable amount of time.

Thus, the experiments were done with protein concentrations in the interval 0.05 - 0.07 mg mL-1 in HEPES buffer supplemented with 200 mM NaCl at pH 7.5. The ellipticity of the protein was monitored from 15 to 80°C (the interval 15 - 70°C is represented for clarity) and the sample was heated at a constant gradient of 1°C min-1.

DO1 Cas9 was dialyzed previous to the experimental study. To compare with CD studies, experiments were carried out using a fresh frozen stock. The experimentally determined fluorescence intensity vs. temperature data was fitted to a sigmoidal Boltzman equation assuming linear initial and final baselines (for either native or denatured states). This equation highly improves the reproducibility of the fit (especially the two relevant parameters, tm and dt) when different wavelengths are monitored for a single experiment.

Considering that the variation of fluorescence intensity with temperature would only give the changes in hydration in the local environment of the TRP residue, the results obtained from CE studies were considered more reliable. Nevertheless, it is important to note that the fluorescence results are qualitatively in line with the ones discussed before regarding the circular dichroism experiments.

On the other hand, fluorescence spectroscopy monitors the intrinsic emission of (mainly) tryptophan (TRP) residues upon excitation at a given wavelength. Both the position (maximum wavelength) and intensity of the emission band depend strongly upon the hydration (degree of exposure of these residues to the aqueous solvent). Therefore, by following the heat-induced change in the fluorescence emission of a protein, the local environment around the TRP residues was monitored. The intrinsic fluorescence of the protein reveals changes in the solvent accessibility of TRP. The denaturation of the protein may be detected following these changes in the fluorescence intensity of the protein as a function of temperature, but the overall process might be obscured when the degree of TRP residues exposure to the solvent is similar in the initial (native) and final state (aggregated), leading to a spectroscopically silent process.

The experiments were done in a concentration interval 0.18 - 0.22 mg.mL-1 in HEPES buffer supplemented with 200 mM NaCl at pH 7.5. The ellipticity of the protein was monitored from 6 to 80°C (the interval 6 - 70°C is presented for clarity) when the sample was heated to a constant gradient of 1 °C·min-1.

### 6.2 RESULTS

### 6.2.1 Protein stability

RNP activity of nuclease of invention is affected when the protein is incubated at warmer temperatures. The loss in activity depends on the time and temperature used in the incubation. After one hour of incubation, the nuclease of the invention DO1 Cas9 is active at 28°C, but it is dramatically affected at 30°C, and it is inactivated when the temperature is 33°C and above, whereas SpCas9 is still active at 35°C (Figure 11). An incubation of 15 minutes at 33°C is enough to abolish the activity of the nuclease of invention, although there is a heavy decrease of it after only 5 minutes (Figure 12). Determination of protein stability within a narrower one-degree temperature increase range showed that nuclease of invention activity can be inactivated at the lower temperature of 31°C with a one-hour incubation whereas SpCas9 is still active at 40°C (Figure 13).

### 6.2.2 Cold-adapted

### Analysis of nuclease structure

An ensemble of nuclease of the invention structural models generated with AlphaFold2 (Figure 14A) showed a large conformational heterogeneity, suggestive of high structural flexibility. The structural flexibility observed in DO1 Cas9 is in line with the general understanding that all the structural factors stabilizing a protein molecule are attenuated in psychrophilic enzymes, both in strength and number (Feller& Gerday. Psychrophilic enzymes: hot topics in cold adaptation. Nature Reviews Microbiology. 2003; 1, 200-208*).*

In general, achieving high activity at low temperatures throughout evolution has involved destabilizing the active site or the whole protein, allowing the catalytic center to be more mobile or flexible in temperatures that tend to freeze molecular motions. Nuclease described herein shows much larger structural fluctuations when compared to SpCas9 or another Cas9 protein of similar length such as GeoCas9 (1087 aas). Large structural fluctuations are evident throughout nuclease described herein, particularly in the HNH catalytic domain and the PAM-interacting region (Figure 14B).

The psychrophilic profile of nuclease of the invention DO1 Cas9 is further supported by predictions of its folding stability (Figure 14C) by structure-based generative models (Matteo et al. Predicting absolute protein folding stability using generative models. BiorXiv. 2024 March 15). When compared to GeoCas9, which is a thermostable Cas9 variant (denaturation temperature (Td) of ~65°C; and to SpCas9, nuclease of the invention exhibits much lower stability, once again consistent with an optimal activity at low temperatures (Figure 14C).

*Determination of thermal stability and activation energy of nuclease using circular dichroism (CD) and fluorescence spectroscopies (FS).*

The initial results obtained by CD and FS clearly indicated that the heat-induced denaturation of the proteins was irreversible leading to aggregation.

### Results from DC

Figure 15 presents the results of the thermal denaturation of the studied proteins monitored using circular dichroism.

The ellipticity of the protein remains fairly stable (increases slightly, in negative values) while the protein is fully populating a given conformational state. As the sample absorbs increasing amounts of energy in the form of heat, the protein starts to unfold, losing some elements of secondary structure leading to an increase in its ellipticity in a sigmoidal fashion until the final (aggregated) state is fully populated. The sigmoidal behaviour is indicative that the kinetically controlled process involves mainly only two states: the initial native state and the final denatured one.

The experimentally determined ellipticity vs. temperature data was fitted to a sigmoidal Boltzman equation assuming linear initial and final baselines (for either native or denatured states). Two main features are relevant: the inflection point of the sigmoidal (the temperature at which the curve turns from concave to convex) that is usually denoted by tm and the slope of the sigmoidal curve t = tm , that is inversely related to dt: a low value of dt denotes a large slope (the sigmoidal is less open and the transition between the two conformational states is completed in a smaller temperature interval). On the contrary, a large value of dt means that the slope at tm is small, the sigmoidal curve looks more spread and the complete transformation of the native into the denatured state needs a larger temperature interval to be completed.

Nuclease of the invention and SpCas9 show a low, and similar, thermal stability. However, regarding dt, the differences give us and idea of the activation energy of the heat-induced denaturation process (Table 6).

For SpCas9, its dt value (1.7) is low meaning that the activation energy is high, and the rate of denaturation increases rapidly with temperature. Denaturation of SpCas9 seems to start slightly below 25°C and is completed when temperature reaches 45oC. In the case of the nuclease described herein, its dt is higher (28) meaning that the activation energy is lower, and denaturation transition seems to start at temperatures slightly above 25°C and increase gradually instead of rapidly.

Results also suggest a longer period in native state below 25°C for nuclease of the invention.

**Table 6. Analysis of thermal denaturation of proteins tested monitored using circular dichroism.**

| | Circular dichroism | | | |
|---|---|---|---|---|
| | tm (°C) | | dt | |
| Protein | Value | Error | Value | Error |
| Nuclease of the invention | 34.2 | 0.4 | 2.8 | 0.4 |
| SpCas9 | 36.8 | 0.5 | 1.7 | 0.3 |

### Results from FS

Figure 16 and Table 7 presents the results of the thermal denaturation experiments using fluorescence. Similar results and conclusions are obtained when analyzing the temperature dependence of the fluorescence intensity of the proteins in term of tm although results are not conclusive for dt due to turbidity issues on the Spcas9 sample. In any case, as observed in Figure 16, resulting sigmoidal curves are different between DO1 Cas9 and SpCas9, with a less pronounced slope (and therefore higher dt) in the case of DO1 Cas9.

**Table 7. Analysis of temperature dependence of the fluorescence intensity of the proteins.**

| | Steady-state fluorescence | | | |
|---|---|---|---|---|
| | tm (°C) | | dt | |
| Protein | Value | Error | Value | Error |
| Nuclease of the invention | 33.9 | 0.5 | **2.5** | 0.7 |
| SpCas9 | 35.9 (*) | 1.0 | 2.7 (*) | 0.8 |

| | | | | |
|---|---|---|---|---|
| *(*): These values may be biased due to the increase in the turbidity of the sample (and therefore on the dispersion of emitted light) due to massive aggregation of the protein upon its denaturation* | | | | |

### Example 7. Evaluation of in vivo nuclease activity in Caenorhabditis elegans

### 7.1 MATERIALS AND METHODS

*Caenorhabditis elegans* hermaphrodites at the Young Adult stage were immobilized on 2% agar pads with halocarbon oil and injected into the germline with the corresponding transformation (microinjection) mix using the XenoWorks Microinjection System (Sutter Instrument) and the Nikon Eclipse Ti-S inverted microscope with Nomarski optics. The final reaction volume was 10 µL, and the mixture was incubated at 20°C for 15 minutes. Afterward, it was centrifuged at 13,000 rpm for 2 minutes to allow the settling of particulate matter. The injection mixes were kept on ice before loading the needles, and any excess was stored at -20°C. The F1 progeny were then separated individually or in pools onto NGM agar plates, allowed to lay F2 progeny, and subsequently genotyped via PCR.

Microinjection mix: 1µl Cas9 (3.1µM), 0.5µl gRNA synthetized by IDT (100µM), 2µl mlc-1:GFP plasmid (130ng/µL), 6.5µl nuclease-free water.

### 7.2. RESULTS

Worms from the progeny (F1) showing fluorescence (from mlc-1:GFP) were singled-out and F2 dumpy mutants were selected for DNA extraction and amplification of the *dpy-10* gene (Figure 17A). PCR amplicons for the *dpy-10* gene in dumpy mutants were sequenced. By doing Sanger sequencing on PCR fragments of *C. elegans dpy-*10 mutants produced by Cas9 (Figure 17B), most of them (7of8) were found to have deletions, most of them being 4bp or 5bp deletions, upstream of the PAM sequence (SEQ ID NO: 22 to SEQ ID NO: 29), and a small insertion (SEQ ID NO: 30) indicating that DO1 Cas9 cleavage site is at position 3bp upstream of the PAM sequence, as expected from the determination of the cut sites *in vitro* (Table 4). SEQ ID NO: 22 is a wild-type animal used as control.

### Example 8. Evaluation of nuclease activity in zebrafish

### 8.1 MATERIALS AND METHODS

Nuclease mRNA (150 ng/µl) and gRNA (150 ng/µl) against the tbxta zebrafish gene were injected in zebrafish embryos, and then incubated a 25°C, 28 °C and 33°C.

Besides using mRNA to express Cas9 in zebrafish embryos, purified protein was used as RNP.

### 8.2 RESULTS

Editing was detected in 42% of embryos at 25°C, and 25% of embryos at 33°C (Figure 18). This experiment suggests that DO1 Cas9 would be more efficient in vivo at lower temperatures than at warmer temperatures.

Thus, Cas9 would be suitable for editing any other living organism, although its natural activity would be low or none in warm-blood animals because of its thermosensitivity.

When purified protein was used as RNP it was also efficient in gene editing, although protein concentration would be optimized for a higher efficiency (Figure 19).

### Example 9. Determination of structure of nuclease and potential versions resulting from mutating key residues.

### 9.1 MATERIALS AND METHODS

The structure of nuclease of the invention was modeled with the alpha fold *(*Jumper et al. Highly accurate protein structure prediction with AlphaFold. Nature. 2021: 596(7873), 583-589*).*

### 9.2 RESULTS

Modeling of the nuclease structure allowed for identifying catalytic residues that can be mutated to produce inactive (H588A) and nickase (D9A) versions of the nuclease (Figure 20).

Moreover, this information would be profitable to improve activity and specificity, and to modify the PAM sequence.

## Claims

1. A ribonucleoprotein complex comprising:
(i) a nuclease, wherein the nuclease comprises an amino acid sequence having a sequence identity of at least 70%, at least 75%, at least 80%, at least 85%, at least 90%, at least 95%, at least 98%, at least 99% or 100% to SEQ ID NO: 1; and
(ii) a guide RNA, wherein the guide RNA is a dual or single guide RNA comprising:
a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3, and
b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4.

2. The ribonucleoprotein complex according to claim 1, wherein the nuclease binds and cleaves the target polynucleotide in a protospacer adjacent motif (PAM) - dependent manner.

3. The ribonucleoprotein complex according to claim 1 or 2, wherein the nuclease cleaves a double-stranded target polynucleotide at the +3 position from PAM leaving blunt ends, or a single-stranded target polynucleotide at the +3 position from PAM.

4. The ribonucleoprotein complex according to claim 2 or 3, wherein the PAM sequence comprises the sequence NRVDHCN wherein N is any base, R is A or G, V is A or C or G, D is G or T or A, H is C or T or A, C is Cytosine, N is any base.

5. The ribonucleoprotein complex according to any one of claims 1 to 4, wherein the nuclease as ribonucleoprotein complex is enzymatically active in a temperature range from 0°C to 38°C.

6. The ribonucleoprotein complex according to any one of claims 1 to 5, wherein the nuclease as ribonucleoprotein complex is enzymatically inactive in a temperature above 38°C.

7. A dual or single guide RNA comprising:
a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3,
b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4.

8. A vector comprising
- a polynucleotide comprising a nucleotide sequence encoding a nuclease, wherein the nuclease comprises an amino acid sequence having a sequence identity of at least 70%, at least 75%, at least 80%, at least 85%, at least 90%, at least 95%, at least 98%, at least 99% or 100% to SEQ ID NO: 1, preferably, the nucleotide sequence encoding the nuclease comprises the sequence SEQ ID NO: 2; and/or
- the dual or single guide RNA according to claim 7.

9. A cell comprising:
(i) the ribonucleoprotein complex according to any one of claims 1 to 6, and/or
(ii) a nuclease comprising an amino acid sequence having a sequence identity of at least 70%, at least 75%, at least 80%, at least 85%, at least 90%, at least 95%, at least 98%, at least 99% or 100% to SEQ ID NO: 1, and/or
(iii) a polynucleotide comprising a nucleotide sequence encoding the nuclease according to (ii), and/or
(iv) the dual or single guide according to claim 7, and/or
(v) the vector according to claim 8.

10. A nuclease comprising an amino acid sequence having a sequence identity of at least 70%, at least 75%, at least 80%, at least 85%, at least 90%, at least 95%, at least 98%, at least 99% or 100% to SEQ ID NO: 1.

11. The nuclease according to claim 10, wherein the nuclease is enzymatically active in a temperature range from 0°C to 38°C.

12. A polynucleotide comprising a nucleotide sequence encoding a nuclease according to claim 10 or 11, preferably, the nucleotide sequence encoding the nuclease comprises the sequence SEQ ID NO: 2.

13. Use of
(i) the ribonucleoprotein complex according to any one of claims 1 to 6, and/or
(ii) the dual or single guide according to claim 7, and/or
(iii) the vector according to claim 8, and/or
(iv) the nuclease according to claim 10 or 11, and/or
(v) the polynucleotide according to claim 12,
for modifying *in vitro* a target nucleic acid sequence that comprises binding, cutting or cleaving specifically a target nucleic acid sequence, wherein the elements (i) to (v) are not use for modifying the germ line genetic identity of human beings.

14. Use of the ribonucleoprotein complex, the dual guide RNA, the single guide RNA, the vector, the nuclease or the polynucleotide according to claim 13, wherein cutting or cleavage of a target nucleic acid sequence is carried out in a temperature range of from 0°C to 38°C.

15. A ribonucleoprotein complex according to any one of claims 1 to 6, and/or a dual or single guide RNA according to claim 7, and/or a vector according to claim 8, and/or a nuclease according to claim 10 or 11, and/or a polynucleotide according to claim 12, for use in therapeutic modifying a target nucleic acid sequence that comprises binding, cutting or cleaving specifically a target nucleic acid sequence.
